# EUROPEAN PATENT SPECIFICATION

(11) **EP 3 418 747 B1**
(45) Date of publication and mention of the grant of the patent: **12.01.2022**
(21) Application number: 17753236.3
(22) Date of filing: 15.02.2017
(51) Int. Cl.: G01N 33/68, C07K 14/47, G01N 33/53

(54) **BLOOD BIOMARKER FOR DETECTING ARTERIOSCLEROSIS**
BLUTBIOMARKER FÜR DEN NACHWEIS VON ARTERIOSKLEROSE
BIOMARQUEUR SANGUIN DE DÉTECTION D'ARTÉRIOSCLÉROSE

(30) Priority: 16.02.2016 JP 2016026494
(43) Date of publication of application: 26.12.2018
(73) Proprietor: Public University Corporation Yokohama City University, Yokohama-shi, Kanagawa 236-0027 (JP); Tosoh Corporation, Yamaguchi 746-8501 (JP)
(72) Inventor: YOKOYAMA, Utako, Yokohama-shi Kanagawa 236-0004 (JP); ISHIKAWA, Yoshihiro, Yokohama-shi Kanagawa 236-0004 (JP); ARAKAWA, Noriaki, Yokohama-shi Kanagawa 236-0004 (JP); MASUDA, Munetaka, Yokohama-shi Kanagawa 236-0004 (JP); ISHIGAMI, Tomoaki, Yokohama-shi Kanagawa 236-0004 (JP); SUZUKI, Shin-ichi, Yokohama-shi Kanagawa 236-0004 (JP); OHTAKE, Norihisa, Ayase-shi Kanagawa 252-1123 (JP); KOBORI, Hiroki, Ayase-shi Kanagawa 252-1123 (JP)
(74) Representative: Mewburn Ellis LLP
(86) International application number: PCT/JP2017/005565
(87) International publication number: WO 2017/141980

(56) References cited:
- EP-A1- 2 159 577
- WO-A1-90/11520
- WO-A1-2006/061690
- WO-A2-01/04264
- JP-A- 2007 121 308
- LAURA S SHANKMAN ET AL: "KLF4-dependent phenotypic modulation of smooth muscle cells has a key role in atherosclerotic plaque pathogenesis", NATURE MEDICINE, vol. 21, no. 6, 18 May 2015 (2015-05-18), pages 628-637, XP055597541, New York ISSN: 1078-8956, DOI: 10.1038/nm.3866
- RYOZO NAGAI et al.: "Smooth Muscle Myosin Heavy Chain Expression in the Arterial Wall; a New Viewpoint for Vascular Pathology", The Japanese Journal of Clinical Pathology, vol. 43, no. 4, April 1995 (1995-04), pages 337-341, XP009513105, ISSN: 0047-1860
- MASANORI AIKAWA et al.: "Diversity of human smooth muscle myosin heavy chains", Juntendo Medical Journal, vol. 40, no. 2, 1994, pages 189-199, XP055538115,

## Description

### TECHNICAL FIELD

The present invention relates to a method for detecting arteriosclerosis using as an indicator the blood myosin heavy chain 11 level, as set out in claim 1.

### BACKGROUND ART

Due to aging of the population and changes in the lifestyle, diseases mainly involving arteriosclerosis, such as coronary artery diseases, peripheral arterial diseases, and aortic aneurysm, have been increasing. In order to improve prognosis in arteriosclerotic diseases, it is necessary to diagnose arteriosclerotic diseases by a non-invasive, relatively simple test and design an appropriate examination and treatment plan.

Examples of the method for non-invasively detecting arteriosclerosis include methods in which the intima media thickness, the presence of plaques, and the like are measured by carotid artery ultrasonography. However, in such methods, diagnosis is made based on observation of the limited region, carotid artery only, and therefore the methods are not necessarily capable of detecting arteriosclerosis in other regions.

Known risk factors for development of arteriosclerosis include diabetes, smoking, hyperlipidemia, and hypertension (Non-Patent Document 1). However, at present, thanks to the progress of pharmacotherapy for hypertension and hyperlipidemia, the risk of arteriosclerosis cannot be judged based solely on these classical risk factors. Since arteriosclerosis itself is asymptomatic, a patient cannot become aware of its progression. Thus, biomarkers capable of diagnosis of arteriosclerosis have been demanded.

In recent years, an increased number of studies have been carried out for biomarkers based on the mechanism of development of arteriosclerosis. Progression of arteriosclerosis is known to involve disorders of vascular endothelial functions, abnormality of the coagulation-fibrinolytic system, inflammation, lipid metabolism abnormality, and the like, and association between proteins related to these factors and acute coronary syndrome has been shown (Non-Patent Document 2). However, since increases in these proteins may also occur due to infections, cancers, traumas, and the like, they are poorly specific to blood vessels, so that arteriosclerosis cannot be diagnosed only by a single protein molecule. In Non-Patent Document 2, not less than 20 factors associated with lipids, metabolism, coagulation, angiogenesis, renal function/inflammation, oxidative stress, and the like have been studied. However, no biomarker better than the classical risk factors has been discovered.

In Patent Document 1, a large number of proteins including tumor necrosis factor-α precursor have been proposed as biomarkers capable of predicting cardiovascular events. The cardiovascular events include various arteriosclerotic diseases such as aortic aneurysm rupture, myocardial infarction, stroke, and peripheral arterial diseases. However, these biomarkers do not diagnose arteriosclerosis itself.

Patent Document 2 discloses blood YKL-40 as a prognostic biomarker for heart diseases caused by atherosclerosis. This biomarker also does not diagnose arteriosclerosis itself. Further, since YKL-40 is found in blood also in cases of cancer and inflammation (Non-Patent Documents 3 and 4), its disease specificity is low.

Patent Document 3 discloses a method for evaluating an arteriosclerotic lesion using as indicators marker proteins whose expression changes along with the progression of the arteriosclerotic lesion. However, since these marker proteins have been identified using arteriosclerosis model animals, whether or not they are capable of evaluation of human arteriosclerotic lesions is unclear. Further, since the method is based on investigation of protein expression levels in the arterial tissue lesion, the method, similarly to carotid artery ultrasonography, is not necessarily capable of detecting the presence of arteriosclerosis in regions other than the region to be examined.

Non-Patent Document 5 discloses that smooth muscle cells (SMCs) contribute to atherosclerotic plaques, that myosin heavy chain 11 (MYH11) is associated with SMCs involved in atherosclerotic lesions, and that KLF4-dependent transitions in SMC phenotype are critical in atherosclerotic lesion pathogenesis.

Patent Document 4 discloses the association of a number of polynucleotides and polypeptides with atherosclerosis, including myosin heavy chain 11 (MYH11).

Patent Document 5 discloses an ex vivo method of diagnosing or predicting an arterial disease, or a risk of arterial disease, in a subject, which method comprises detecting a mutation in the MYH11 gene or protein, wherein said mutation is indicitive of an arterial disease or a risk of arterial disease.

Patent Document 6 discloses a method for evaluating the presence or level of an arteriosclerotic disease in a mammal, or a method for evaluating the prophylactic or therapeutic effect on an arteriosclerotic disease in a mammal, which is characterized by detecting a soluble LR11 in a sample collected from the mammal.

### PRIOR ART DOCUMENT(S)

### PATENT DOCUMENT(S)

Patent Document 1: JP 2010-534852 A
Patent Document 2: JP 2011-510308 A
Patent Document 3: WO 2012/067180
Patent Document 4: WO 2001/04264 A2; Patent Document 5: WO 2006/061690; Patent Document 6: EP 2159577A1

### NON-PATENT DOCUMENT(S)

Non-Patent Document 1: Anderson KM. et al., Am Heart J, 1991; 121: 293-8
Non-Patent Document 2: Blankengerg S. et al., Circulation, 2010; 121: 2388-97
Non-Patent Document 3: Kring TS., et al., Biomark Cancer, 2015; 7: 57-61
Non-Patent Document 4: Ural UM., et al., Clin Exp Obstet Gynecol, 2015; 42: 495-7
Non-Patent Document 5: Shankman LS., et al., Nat Med, 2015; 21: 628-37

### SUMMARY OF THE INVENTION

### PROBLEMS TO BE SOLVED BY THE INVENTION

An object of the present invention is to provide novel means capable of detecting an arteriosclerotic lesion in a body by a less invasive, simple method.

### MEANS FOR SOLVING THE PROBLEMS

As a result of intensive study, the present inventors discovered that the blood myosin heavy chain 11 level in arterial disease patients having a severe arteriosclerotic lesion is much higher than that in healthy individuals, thereby completing the present invention.

That is, the present invention provides a method for detecting arteriosclerosis, comprising measuring the myosin heavy chain 11 level in a blood sample isolated from a subject, wherein a myosin heavy chain 11 level higher than that in a healthy individual is indicative of the presence of arteriosclerosis in the subject. The present invention also provides the use of an anti-myosin heavy chain 11 antibody or an antigen-binding fragment as set out in claim 2.

### EFFECT OF THE INVENTION

The present invention revealed, for the first time, that myosin heavy chain 11 can be used as a blood biomarker reflecting the presence of arteriosclerosis in a body. Since this allows detection of arteriosclerosis by a blood test, a less invasive, simple test can be carried out. Further, since the method, unlike carotid artery ultrasonography, is not based on examination of a particular region only, it is expected to reflect the conditions of blood vessels in the whole body. By detecting arteriosclerosis itself by the present invention, the risk of development of an arteriosclerotic disease can be known before the development. This can provide a chance for promoting self-improvement of the lifestyle, leading to prevention of development of the disease and contributing to reduction of the medical cost. Moreover, in a patient at an early stage of development of an arteriosclerotic disease wherein the patient hardly has subjective symptoms, knowing of the presence of severe arteriosclerosis by the method of the present invention may lead to early detection of the disease, which enables design of a treatment plan at an earlier phase, contributing to improvement of the life prognosis of the patient. By the present invention, data useful for detection of the presence of an arteriosclerotic lesion can be provided, so that evaluation and diagnosis of the arteriosclerosis itself by a physician can be greatly assisted.

### BRIEF DESCRIPTION OF THE DRAWINGS

### BRIEF DESCRIPTION OF THE DRAWINGS

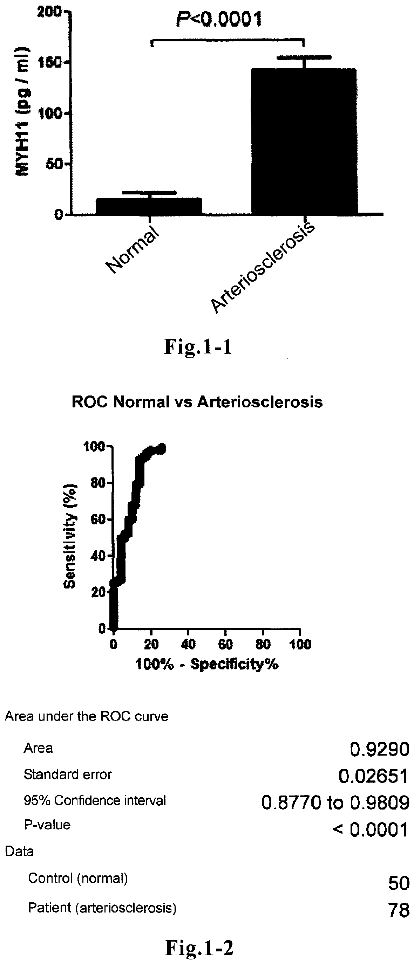

Fig. 1-1 shows the result of comparison of the measured value of the blood myosin heavy chain 11 level between a normal group and an arteriosclerosis patient group. A significant difference between the groups was found at P<0.0001 (Welch's t-test).
Fig. 1-2 shows the result of evaluation, by ROC analysis, of the capacity of the blood myosin heavy chain 11 level to detect the arteriosclerosis patient group.
Fig. 2-1 shows the result of comparison of the measured value of the blood myosin heavy chain 11 level among the following three groups: a normal group, an arteriosclerosis patient group that received PCI, and an arteriosclerosis patient group that received EVT. *** indicates a significant difference from the normal group at P<0.0001 (Welch's t-test).
Fig. 2-2 shows the result of evaluation, by ROC analysis, of the capacity of the blood myosin heavy chain 11 level to detect each arteriosclerosis patient group.
Fig. 3-1 shows the result of comparison of the measured value of the blood myosin heavy chain 11 level among the following three groups: a Japanese normal group, an arteriosclerosis patient group that received PCI, and an arteriosclerosis patient group that received EVT. *** indicates a significant difference from the normal group at P<0.0001 (Welch's t-test).
Fig. 3-2 shows the result of evaluation, by ROC analysis, of the capacity of the blood myosin heavy chain 11 level to detect each arteriosclerosis patient group.
Fig. 4 shows the result of investigation of correlation between the blood MYH11 level and the blood CRP level in 46 arteriosclerosis patients.
Fig. 5 shows the result of evaluation, by ROC analysis, of the capacity of the blood CRP level to detect each arteriosclerosis patient group.
Fig. 6 shows the result of investigation of correlation between the blood MYH1 1 level and the Brinkman index in Japanese healthy individuals, PCI patients, and EVT patients.
Fig. 7 shows the result of measurement of the molar concentrations of two target peptides (ALELDPNLYR and LEVNMQALK) of MYH11 present in a sample using internal standard peptides as indices.
Fig. 8 shows the result of comparison of the blood MYH11 level before the intervention, immediately after the intervention, or within several days after the intervention between a surviving group and a death group (observation period, 1 to 2.5 years) of arteriosclerosis patients who received PCI or EVT.
Fig. 9-1 shows alignment of the amino acid sequences of four isoforms of myosin heavy chain 11.
Fig. 9-2 shows alignment of the amino acid sequences of four isoforms of myosin heavy chain 11 (continued from Fig. 9-1).
Fig. 9-3 shows alignment of the amino acid sequences of four isoforms of myosin heavy chain 11 (continued from Fig. 9-2).
Fig. 9-4 shows alignment of the amino acid sequences of four isoforms of myosin heavy chain 11 (continued from Fig. 9-3).
Fig. 9-5 shows alignment of the amino acid sequences of four isoforms of myosin heavy chain 11 (continued from Fig. 9-4).

### MODE FOR CARRYING OUT THE INVENTION

For myosin heavy chain, isoforms such as 5, 7, 10, and 11 are known. Among these, myosin heavy chain 11 (Gene ID 4629) is the target of the present invention. Myosin heavy chain 11 is an isoform specifically expressed in smooth muscle, and also called smooth muscle myosin heavy chain. Two myosin heavy chain 11 molecules expressed in smooth muscle are combined with two pairs of myosin light chain molecules, each pair consisting of the same type of the light chain, to form a hexameric smooth muscle myosin molecule.

It is known that myosin heavy chain 11 has four isoforms, SM1A, SM1B, SM2A, and SM2B (GenBank Accession Nos. NP_002465.1, NP_001035203.1, NP_074035.1, and NP 001035202.1, whose amino acid sequences are shown in SEQ ID NOs:1 to 4, respectively), which isoforms vary mainly in the C-terminal sequence. The isoform of myosin heavy chain 11 to be measured in the present invention is not limited. All four isoforms may be measured, or only part of the isoforms may be measured.

The blood myosin heavy chain 11 level can be investigated by measuring myosin heavy chain 11 protein or a fragment thereof in a blood sample. Since myosin heavy chain 11 is not a secretory protein, it is thought to be present also in a fragmented form in blood. The myosin heavy chain 11 protein or the fragment thereof to be measured includes one present in blood as a monomer and one present in blood in a state where it is bound to or associated with another protein or the like (for example, one present in blood in a state where it constitutes a complete or partially-degraded smooth muscle myosin molecule). Thus, the term "myosin heavy chain 11" used to refer to a blood biomarker for detection of arteriosclerotic lesion includes not only the full-length myosin heavy chain 11 protein present in blood in a monomeric state and a partial fragment thereof, but also myosin heavy chain 11 protein and a partial fragment thereof in a state where they are bound to or associated with (an)other protein(s) or protein fragment(s).

The blood sample may be serum or plasma. Plasma may be preferably used.

The arteriosclerosis to be detected in the present invention includes arteriosclerosis at any stage between early stage and advanced, late stage. Arteriosclerosis can be pathologically classified into the following three types: atherosclerosis, arteriolosclerosis, and medial calcification. The arteriosclerosis to be detected in the present invention includes these three types. A typical example of the arteriosclerosis to be detected is atherosclerosis.

Examples of diseases caused by arteriosclerosis (arteriosclerotic diseases) include ischemic heart diseases (angina pectoris, myocardial infarction), aortic aneurysm (thoracic/abdominal aortic aneurysm, iliac aneurysm, and the like), arteriosclerosis obliterans, aortic stenosis, and cerebrovascular diseases (cerebral infarction, stroke). By detecting arteriosclerosis itself by the present invention, the risk of development of such diseases can be known before their development. Thus, self-improvement of the lifestyle can be promoted, which leads to prevention of the development of the arteriosclerotic diseases. For subjects in whom severe arteriosclerosis is detected, early detection and early treatment of the diseases become possible by carrying out detailed examination and subsequent careful follow-up.

The means for measuring the blood myosin heavy chain 11 level is not limited as long as it is capable of measuring a polypeptide. Examples of the method for measuring a polypeptide include immunoassays and mass spectrometry. Immunoassays may be preferably used for the measurement of the myosin heavy chain 11 level in the present invention since they do not require a large-scale apparatus and can be carried out by a simple measurement operation. Antibodies capable of detecting myosin heavy chain 11 are known, and there are commercially available products of such antibodies. A commercially available anti-myosin heavy chain 11 antibody is typically a non-human animal polyclonal or monoclonal antibody prepared with a non-human animal such as mouse or rabbit. Since amino acid sequences of myosin heavy chain 11 and base sequences encoding them are known as described above, an anti-myosin heavy chain 11 antibody that specifically recognizes myosin heavy chain 11 may be prepared by a conventional hybridoma method and may then be used.

The anti-myosin heavy chain 11 antibody may be a polyclonal antibody or a monoclonal antibody. An antiserum may be used as a polyclonal antibody. These antibodies may be prepared by well-known conventional methods.

More specifically, an anti-myosin heavy chain 11 polyclonal antibody can be obtained by, for example: immunizing a non-human animal with a myosin heavy chain 11 protein or partial fragment thereof prepared by chemical synthesis or genetic engineering, together with an adjuvant when appropriate; obtaining an antiserum from blood collected from the immunized animal; and then purifying the polyclonal antibody (non-human animal anti-myosin heavy chain 11 polyclonal antibody) from the antiserum. Usually, in order to increase the antibody titer in the immunized animal, the immunization is carried out multiple times over a period of several weeks. The antibody in the antiserum can be purified by, for example, fractionation by ammonium sulfate precipitation, fractionation by anion chromatography, or affinity column purification.

An example of well-known methods for preparation of a monoclonal antibody is the hybridoma method. More specifically, a non-human animal anti-myosin heavy chain 11 monoclonal antibody can be obtained by, for example, collecting antibody-producing cells such as spleen cells or lymphocytes from a non-human animal immunized as described above; fusing these cells with myeloma cells to prepare hybridomas; selecting a hybridoma producing an antibody that binds to myosin heavy chain 11 protein; growing the selected hybridoma; and then obtaining the non-human animal anti-myosin heavy chain 11 monoclonal antibody from the culture supernatant.

The immunoassays themselves are well known in the art. Immunoassay methods can be classified based on the reaction mode into the sandwich method, competition method, agglutination method, Western blotting method and the like, or can be classified based on the label into the enzyme immunoassay, radioimmunoassay, fluorescence immunoassay and the like. In the present invention, any immunoassay method capable of quantitative detection may be used. Preferred Examples of such a method include, but are not limited to, sandwich methods such as sandwich ELISA.

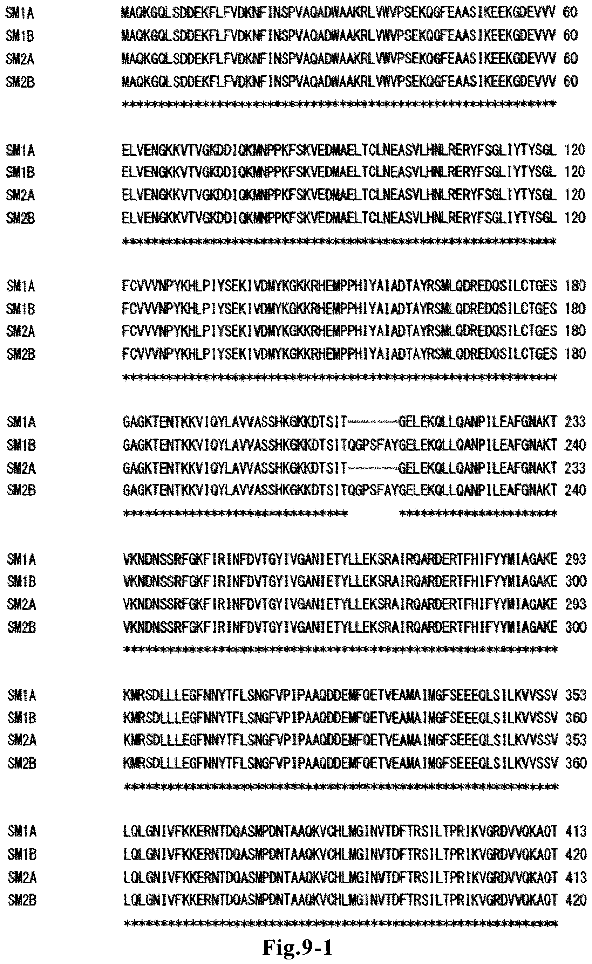

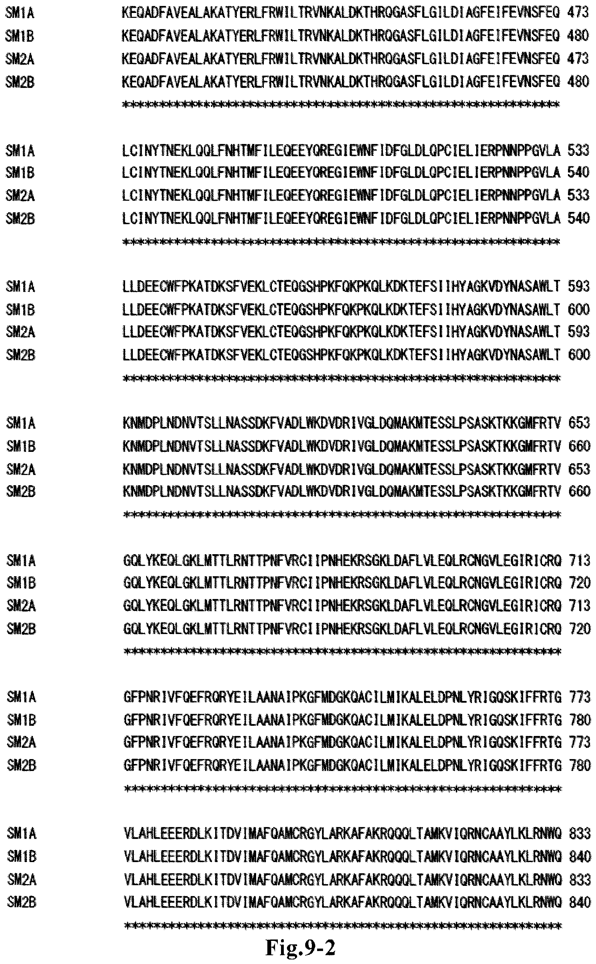

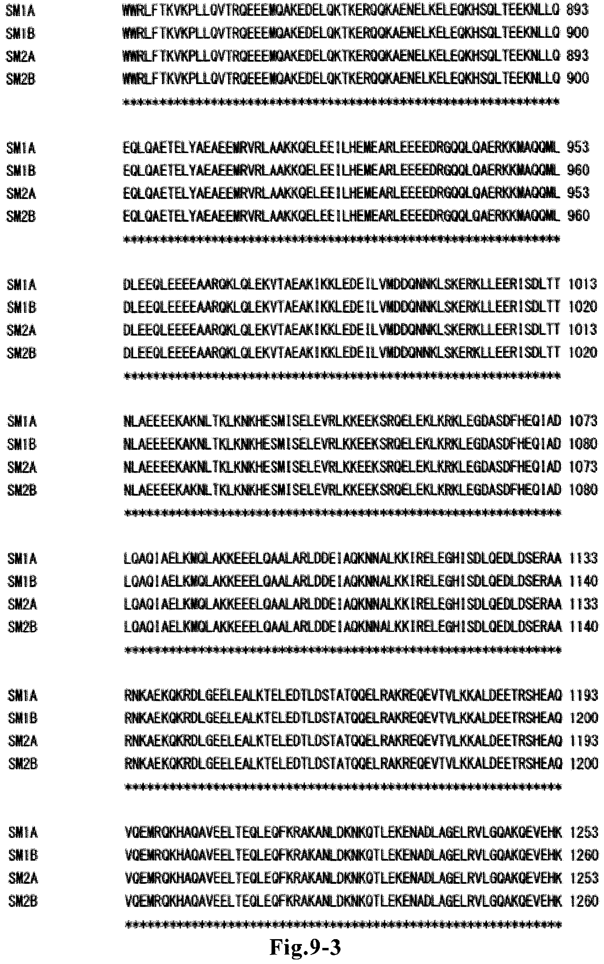

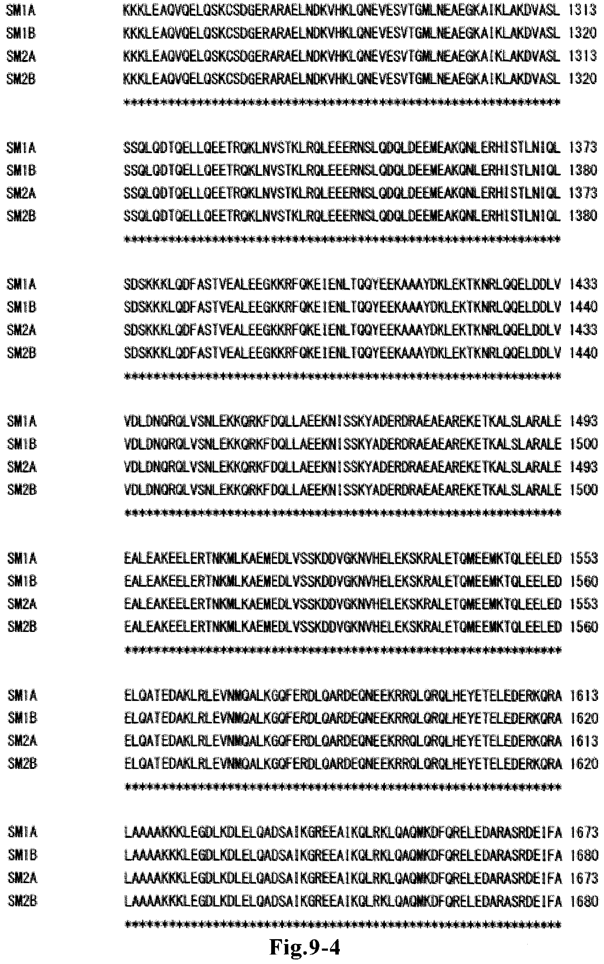

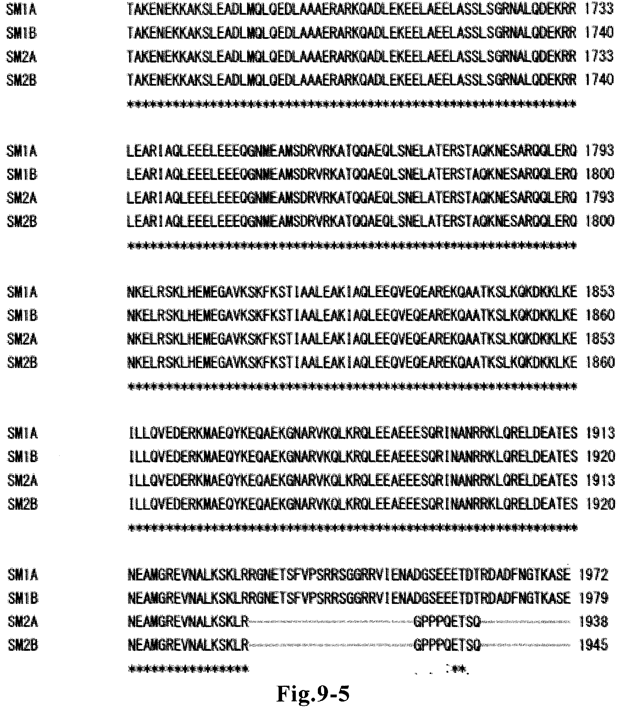

As described above, two myosin heavy chain 11 molecules are contained in a smooth muscle myosin molecule. Such a complex containing two myosin heavy chain 11 protein molecules or two fragments thereof can be measured by a sandwich method using the same antibodies that recognize the same site of myosin heavy chain 11. When a monomeric myosin heavy chain 11 protein or a fragment thereof, or a complex containing just one myosin heavy chain 11 molecule is to be measured by a sandwich method, two kinds of antibodies that recognize different sites of myosin heavy chain 11 may be used. When a polyclonal antibody is employed, the same polyclonal antibody can be used both as a labeled antibody and as a solid-phase antibody to measure the protein including the monomeric molecule and its fragments. As described above, four isoforms of myosin heavy chain 11 are known. All four isoforms can be comprehensively measured by using an antibody that recognizes a region commonly shared by the four isoforms. By using antibodies specific to the four isoforms, respectively, the isoforms can be separately measured, or a particular isoform(s) can be measured. As shown in Figs. 9-1 to 9-5, the regions of amino acid positions I to 211 and 219 to 1936 of SEQ ID NO:4 are regions that are commonly shared by the four isoforms shown in SEQ ID NOs:1 to 4. By using these regions or a part of these regions as an immunogen in preparation of the antibody, an antibody reactive to all four isoforms can be prepared. The region of amino acid positions 212 to 219 of SEQ ID NO:4 is a region specific to SM1B and SM2B; the region of amino acid positions 1937 to 1945 of SEQ ID NO:4 is a region specific to SM2A and SM2B; and the region of amino acid positions 1930 to 1972 of SEQ ID NO:1 is a region specific to SM1A and SM1B. Accordingly, by appropriate combination of antibodies that recognize these regions or partial regions thereof, the isoforms can be separately measured, or a particular isoform(s) can be measured.

In the present invention, an antibody that recognizes the C-terminal region of myosin heavy chain 11 protein may be typically preferably used. However, an antibody that recognizes the N-terminal region may be used, or an antibody that recognizes the C-terminal region and an antibody that recognizes the N-terminal region may be used in combination. In the present invention, the N-terminal region means a region in the N-terminal side including the head portion, which is constituted by the motor domain and the regulatory domain, and part of the tail portion having a coiled-coil structure; and the C-terminal region means a region in the C-terminal side including a major part of the tail portion having a coiled-coil structure. More specifically, for example, in the amino acid sequence of SEQ ID NO:4, a region from around the amino acid position 840 to the N-terminus is the N-terminal region, and a region from around the amino acid position 840 to the C-terminus is the C-terminal region. The antibody that recognizes the C-terminal region means an antibody which has its epitope in the the C-terminal region of myosin heavy chain 11 protein and binds to a site in the C-terminal region. The term "antibody that recognizes the N-terminal region" can be defined in the same manner.

The immunoassays themselves are well known, and do not need to be explained in the present description. Briefly, in a sandwich immunoassay, for example, an antibody that binds to myosin heavy chain 11 is immobilized on a solid phase (immobilized antibody), and then reacted with a sample. After washing, the solid phase is reacted with a labeled antibody prepared by adding a label to an antibody that binds to myosin heavy chain 11 at a site which is the same as or different from the site to which the immobilized antibody binds. After washing, the labeled antibody bound to the solid phase is measured. Each of the immobilized antibody and the labeled antibody may be either a polyclonal antibody or a monoclonal antibody. In the immunoassay, an antigen-binding fragment(s) of the antibody(ies) may be used instead of the antibody(ies).

The "antigen-binding fragment" means an antibody fragment which maintains the binding capacity to the corresponding antigen (antigen-antibody reactivity) that the original antibody has, such as the Fab fragment or F(ab')₂ fragment of immunoglobulin. It is well known that such antigen-binding fragments can also be used in immunoassays, and the fragments are useful similarly to their original antibodies. Fab fragments and F(ab')₂ fragments can be obtained, as is well known, by treating antibodies with a protease such as papain or pepsin. The antigen-binding fragment is not limited to Fab fragments and F(ab')₂ fragments, and may be any fragment maintaining the binding capacity to the corresponding antigen. It may also be one prepared by a genetic engineering method. An antibody prepared by expressing its single chain fragment of variable region (scFv) in *E. coli* by a genetic engineering method may also be used. The method for preparing scFv is also well known. scFv can be prepared by: extracting mRNA from a hybridoma prepared as described above; preparing single-stranded cDNA therefrom; performing PCR using primers specific to the H-chain and L-chain of immunoglobulin to amplify the immunoglobulin H-chain gene and L-chain gene; linking these genes together via a linker; introducing the resultant to a plasmid vector after adding appropriate restriction sites thereto; transforming *E*. *coli* with the resulting plasmid vector; and then recovering scFv from the transformed *E. coli.* Such an scFv is also included in the "antigen-binding fragment".

Measurement of the labeled antibody can be carried out by measuring a signal from the labeling substance. The method for measuring the signal is appropriately selected depending on the type of the labeling substance. For example, when an enzyme label is employed, the substrate of the enzyme may be added into the reaction system, and the amount of coloring or luminescence caused by the enzyme reaction may be measured using an absorptiometer or a luminometer. Myosin heavy chain 11 in a sample can be quantified by beforehand performing an immunoassay of standard samples containing myosin heavy chain 11 at various known concentrations using an anti-myosin heavy chain 11 antibody or an antigen-binding fragment thereof, plotting the correlation between the amount of signal from the label and the myosin heavy chain 11 concentration in each standard sample to prepare a calibration curve, carrying out the same operation for a sample whose myosin heavy chain 11 concentration is unknown to measure the amount of signal from the label, and then applying the measured value to the calibration curve.

In the case where both of the immobilized antibody and the labeled antibody are monoclonal antibodies, whether the combination of the monoclonal antibodies is desirable or not can be easily determined by actually performing the immunoassay. If desired, whether the antibodies recognize different epitopes or not may be determined by identifying the site recognized by each antibody. The identification of the site recognized by each antibody can be carried out by a conventional method well known in the art. Briefly, for example, myosin heavy chain 11, which is the corresponding antigen, is partially digested with a protease such as trypsin. A solution of the partial digestion product is then passed through an affinity column to which the antibody whose site of recognition is to be determined is bound, thereby allowing the digestion product to be bound to the column. Thereafter, the bound digestion product is eluted and subjected to conventional mass spectrometry to identify the site recognized by the antibody.

A blood myosin heavy chain 11 level higher than that of a healthy individual is indicative of the presence of arteriosclerosis. By measurement of the blood myosin heavy chain 11 level, data helpful for detection of arteriosclerosis can be provided. The term "detection of arteriosclerosis" includes detection of severe arteriosclerosis. When the measured value of the blood myosin heavy chain 11 level in a subject is higher than that in a healthy individual, it can be judged that the subject may have an arteriosclerotic lesion. Furthermore, the higher the blood myosin heavy chain 11 level, the higher the possibility of the presence of an arteriosclerotic lesion in the body of the subject can be judged to be, or the higher the severity of the arteriosclerosis can be judged to be. The terms "arteriosclerosis with high severity" and "severe arteriosclerosis" include a state where the number of arteriosclerotic lesions present in the body is large, and a state where the degree of progression of arteriosclerotic lesions present in the body is high. The terms "arteriosclerosis with a high degree of progression" and "advanced arteriosclerosis" mean arteriosclerosis at a level at which a stenosis of not less than 50%, for example, not less than 60%, not less than 70%, or not less than 75% is found by angiography. The rate of stenosis as observed by angiography represents, as is well known in the art, to what extent the blood vessel is narrowed as compared to the diameters of the blood vessel at sites upstream and downstream of the stenosis, which sites are thought to be in a healthy state. For example, 75% stenosis means that the diameter of the imaged site is 25% relative to the blood vessel diameters at sites upstream and downstream of the imaged site, which are taken as 100%.

Whether or not the blood myosin heavy chain 11 level measured is higher than that in a healthy individual can be judged by, for example, employing a healthy reference value determined from blood myosin heavy chain 11 levels measured in a large number of healthy individuals as a cut-off value, and comparing the measured level with the cut-off value. Healthy individuals are individuals who are free of arteriosclerosis, and individuals having symptoms of arteriosclerotic diseases are of course excluded from the healthy individuals. The healthy individuals may be selected based on self-declaration by individuals free of arteriosclerosis. Or, for example, individuals free of lifestyle-related diseases known to be risk factors for arteriosclerosis, such as hyperlipidemia, hypertension, diabetes, and obesity, as well as free of arteriosclerotic diseases may be selected. In the selection of healthy individuals, the absence of the smoking habit, the family history of arteriosclerotic diseases, etc. may also be further taken into account. The healthy reference value may be, for example, the average of blood myosin heavy chain 11 levels in healthy individuals, or the value obtained by adding the standard error to the average. When the measured value of the blood myosin heavy chain 11 level in a subject is higher than the cut-off value, there is a possibility that the subject has an arteriosclerotic lesion. The higher the measured value, the higher the possibility of the presence of the arteriosclerotic lesion can be judged to be, or the higher the severity of the arteriosclerosis can be judged to be. The cut-off value may be set according to age (for example, cut-off values for subjects under 30 years old, in the 30s, in the 40s, in the 50s, in the 60s, and in the 70s or older may be set, respectively).

The present invention can also be carried out for the purpose of detecting especially severe arteriosclerosis that has become advanced to a level at which therapeutic treatment is required. In such a case, a severe arteriosclerosis reference value may be determined based on blood myosin heavy chain 11 levels measured in a group of patients who have developed a severe arteriosclerotic disease such as a coronary artery disease or a peripheral arterial disease. As a severe arteriosclerosis reference value, the average of measured values of the blood myosin heavy chain 11 level in the said group of patients, the value obtained by subtracting the standard error from the average, or the like may be employed. When the measured value of a subject is close to or exceeds the severe arteriosclerosis reference value, it can be judged that the subject may have especially severe arteriosclerosis. By carrying out a detailed examination for such a subject as soon as possible, and carrying out an immediate therapeutic treatment if abnormality is found, or carefully following up, early detection and early treatment of arteriosclerotic disease become possible.

Use of an anti-myosin heavy chain 11 antibody or an antigen-binding fragment is provided. As described above, the anti-myosin heavy chain 11 antibody may be an antibody that recognizes a region commonly shared by the four isoforms and can comprehensively measure all the four isoforms, or may be an isoform-specific antibody/antibodies that separately recognize(s) each of the four isoforms. Use of an antibody that recognizes the C-terminal region of myosin heavy chain 11 protein is provided. The antibody can also recognize the N-terminal region.

The anti-myosin heavy chain 11 antibody may be a labeled antibody and an immobilized antibody. The labeled antibody and the immobilized antibody may be the same anti-myosin heavy chain 11 antibody or antigen-binding fragment thereof, or may be different anti-myosin heavy chain 11 antibodies or antigen-binding fragments thereof.

### EXAMPLES

The present invention is described below in more detail by way of Examples. However, the present invention is not limited to the Examples below.

### 1. Quantification of Blood Myosin Heavy Chain 11 by Sandwich ELISA (Part 1)

### <Materials and Methods>

The quantification was carried out for patients who developed an arterial disease such as stenosis, infarction, or aneurysm in a coronary artery, aorta, or peripheral blood vessel due to progression of arteriosclerosis and received catheterization or stenting. The presence of an arteriosclerotic lesion in each patient was confirmed by angiography (confirmation of stenosis or infarction) or CT (confirmation of calcification). Patients whose arteriosclerosis could not be confirmed were excluded. Plasma was separated from blood collected from each patient before the intervention, immediately after the intervention, or within several days after the intervention, and the myosin heavy chain 11 (MYH11) level in the plasma was measured to obtain the blood MYH11 level. Commercially available products of normal plasma samples from 50 individuals (including Caucasians, Hispanics, and Blacks) were purchased and used as negative controls.

The measurement of the MYH11 level in plasma was carried out by sandwich ELISA using a Human Myosin Heavy Chain 11 ELISA Kit (CUSABIO), which uses anti-MYH11 antibodies as an immobilized antibody and as a labeled antibody. HRP was used as the label for the anti-MYH11 antibody, and TMB was used as a substrate for the detection. Coloring was detected using a luminometer. A calibration curve was prepared using standard samples containing smooth muscle myosin at various concentrations. By application to this calibration curve, the MYH11 concentration in each sample was calculated. As the smooth muscle myosin, a recombinant protein included in the above-mentioned kit (CUSABIO) was used.

To identify the region recognized by the anti-MYH11 antibody used in this study, the full length of the MYH11 isoform SM2B (SEQ ID NO:4) was roughly equally divided into two parts to prepare an N-terminal fragment (R1) and a C-terminal fragment (R2), and reactivity of the antibody with each fragment was investigated by ELISA. As a result, the antibody was not reactive with R1, and was reactive only with R2. It was thus confirmed that this antibody recognizes a certain site in the C-terminal half region of MYH11 (in the region of amino acid positions 950 to 1945 of SEQ ID NO:4). Regarding the immobilized antibody in the ELISA kit, it was found to be reactive with the two different fragments of MYH11, and therefore assumed to be a polyclonal antibody.

### <Results>

Fig. 1-1 shows the result of measurement of the blood MYH11 level in 50 normal individuals and a total of 78 patients with arteriosclerosis. Compared to the normal group, the arteriosclerosis patient group clearly showed higher blood MYH11 levels.

Fig. 1-2 shows the result of evaluation, by ROC analysis, of the capacity of the blood MYH11 level to detect the arteriosclerosis group. This result demonstrated a significantly strong association between the MYH11 concentration in plasma, which is an independent variable, and the outcome, that is, the presence/absence of arteriosclerosis, which is a dichotomous variable. The area under the ROC curve (AUC) was 0.9290. AUC takes a value of from 0.5 to 1.0. An AUC value of from 0.5 to 0.7 is judged as "low accuracy"; an AUC value of from 0.9 to 0.7 is judged as "moderate accuracy"; and an AUC value of from 0.9 to 1.0 is judged as "high accuracy". In the present case, the AUC value was over 0.9. It was thus shown that diagnosis of arteriosclerotic lesion using the blood MYH11 level as an indicator is highly useful.

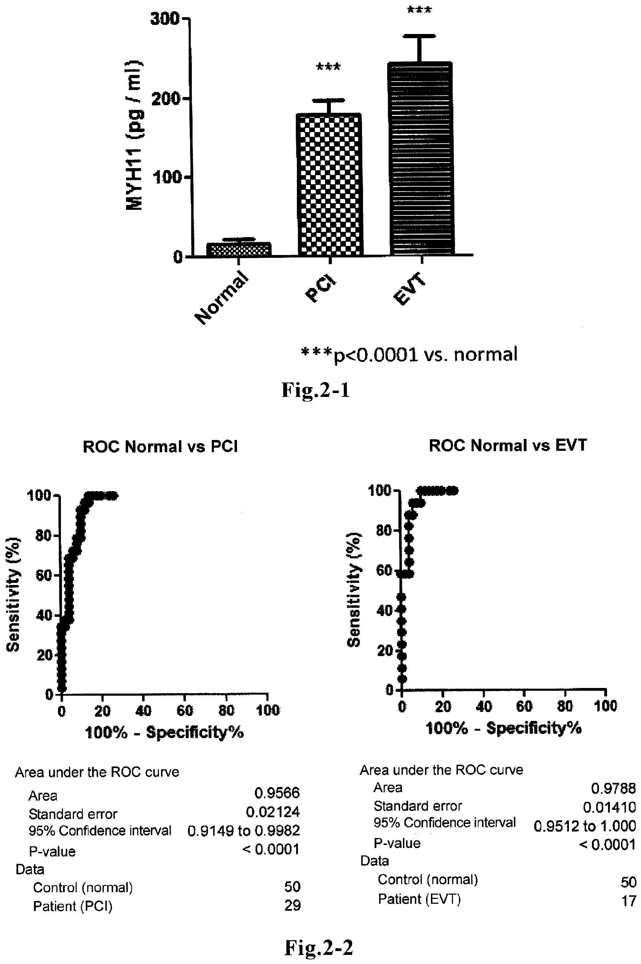

The measured values of the blood MYH11 level of 29 patients who received percutaneous coronary intervention (PCI) and 17 patients who received peripheral artery endovascular treatment (EVT) in the group of arteriosclerosis patients were compared with that of the normal group. The result is shown in Fig. 2-1. The patients in these patient groups had highly advanced arteriosclerosis that required therapeutic treatment for a peripheral artery such as a lower limb artery or for a coronary artery. It could be confirmed that the blood MYH11 level is especially high in such cases of very severe arteriosclerotic lesions. Fig. 2-2 shows the result of evaluation, by ROC analysis, of the capacity of the blood MYH11 level to detect each arteriosclerosis patient group. The AUC value was as high as more than 0.95 in both groups.

### 2. Quantification of Myosin Heavy Chain 11 in Plasma by Sandwich ELISA (Part 2)

### <Materials and Methods>

As arteriosclerosis samples, plasma samples derived from 29 patients to which PCI was applied (69.5 ± 1.4 years old; BMI, 22.4 ± 0.5; male:female = 26:3) and 17 patients to which EVT was applied (72.4 ± 1.4 years old; BMI, 23.6 ± 1.1; male:female = 13:4) out of the arteriosclerosis patient plasma samples in the above-described Section 1 were used. As normal samples, plasma samples from 40 Japanese healthy individuals free of arteriosclerosis and its risk factors (70.4 ± 0.6 years old; BMI, 23.3 ± 0.2; male:female = 33:7) were used. Sandwich ELISA was carried out using the same ELISA kit manufactured by CUSABIO as in the Section 1.

### <Results>

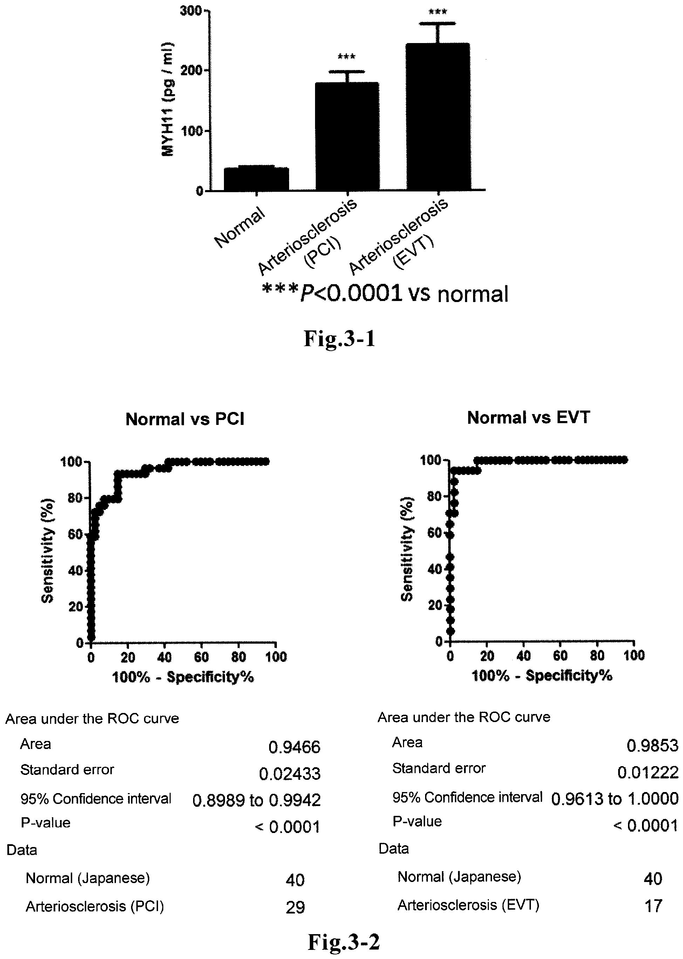

The measured values of the blood MYH11 level of the arteriosclerosis patients to which PCI was applied and the arteriosclerosis patients to which EVT was applied were compared with that of the 40 normal Japanese individuals. The result is shown in Fig. 3-1. Fig. 3-2 shows the result of evaluation, by ROC analysis, of the capacity of the blood MYH11 level to detect each arteriosclerosis patient group. Also in the comparison with the Japanese healthy individuals, the arteriosclerosis patients showed significantly higher blood MYH11 levels, and AUC values were as high as more than 0.9.

### 3. Study of Diagnostic Performance of Blood Myosin Heavy Chain 11 Level for Arteriosclerosis

### (1) Comparison of Diagnostic Performance with CRP

C-reactive protein (CRP) is a marker for prediction of cardiovascular events, and it is known that the blood CRP value increases also in cases of arteriosclerosis (Blankengerg S, et al., Circulation 2010; 121: 2388-97). For evaluation of the diagnostic performance of the blood myosin heavy chain 11 level for arteriosclerosis, the plasma CRP level was investigated for the same arteriosclerosis patients (29 PCI patients and 17 EVT patients) and Japanese healthy individuals as in the Section 2.

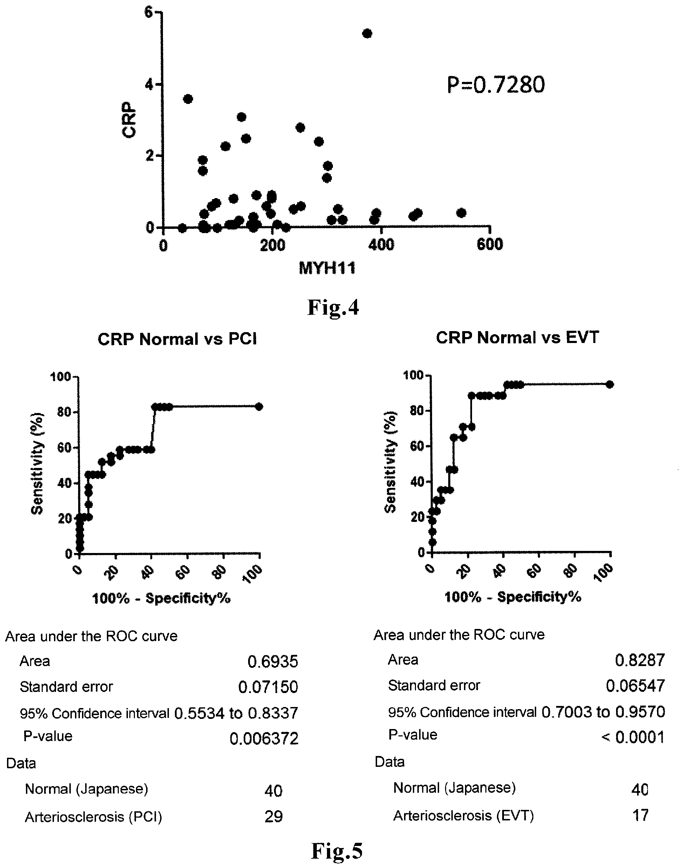

Fig. 4 shows the result of investigation of correlation between the blood MYH11 level and the blood CRP level in the 46 arteriosclerosis patients. It could be confirmed that there is no correlation between them, and that they are factors independent from each other.

Fig. 5 shows the result of evaluation, by ROC analysis, of the capacity of the blood CRP level to detect each arteriosclerosis patient group. The AUC value was as low as less than 0.9 in both groups, and it could thus be confirmed that the diagnostic capacity of CRP for arteriosclerosis is lower than that of MYH11.

### (2) Association with Arteriosclerosis Risk Factors

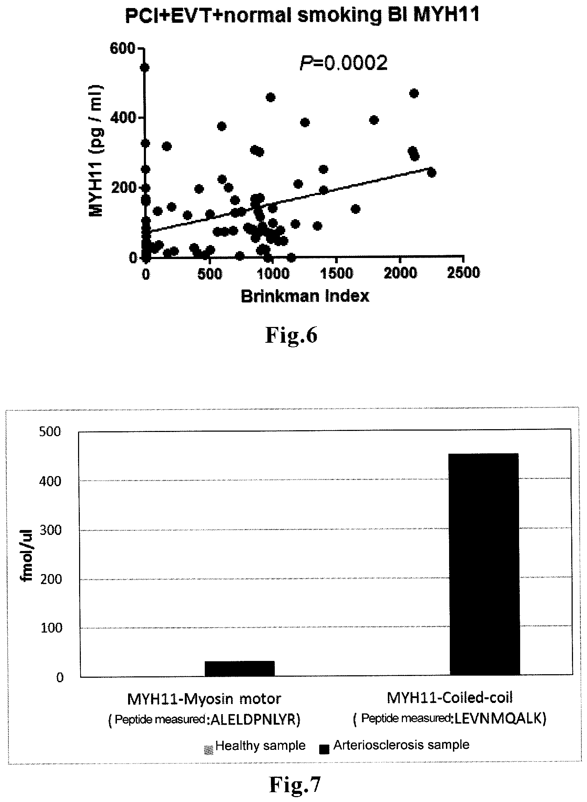

Smoking is one of the major risk factors for arteriosclerosis, and correlation between smoking and arteriosclerosis is known. Correlation between the plasma MYH11 level and the Brinkman index (number of cigarettes smoked per day × number of years of smoking) was investigated in the Japanese healthy individuals, the PCI patients and the EVT patients, and as a result, as shown in Fig. 6, a correlation was found between them. Since slow progression of arteriosclerosis occurs with aging even in healthy individuals, it is thought that the healthy individual population also includes individuals with very mild arteriosclerosis. The PCI patients and the EVT patients are severe cases of arteriosclerosis, which require therapeutic treatment. The data shown in Fig. 6 suggest that even mild arteriosclerosis can be detected based on the blood MYH11 level.

### 4. Analysis of Dynamics of Myosin Heavy Chain 11 in Blood

### <Materials and Methods>

### (1) Preparation of Antibody-immobilized Microparticles

Magnetic microparticles on which an antibody is to be immobilized (Dynabeads (registered trademark) M-280 Tosylactivated; Thermo Fisher Scientific) were suspended in a buffer described in the protocol, and Anti-MYH11 Human (HPA015310; Cosmo Bio Co., Ltd.) was mixed with the resulting suspension, followed by allowing immobilization of the antibody at 37°C for 17 hours. After the immobilization of the antibody, the antibody-immobilized microparticles were suspended in 0.05 mol/L Tris-HCl buffer (pH 8.0) supplemented with 10% Blockmaster CE510 (JSR), and the resulting suspension was incubated at 37°C for 5 hours to perform blocking, thereby obtaining anti-MYH11 antibody-immobilized magnetic microparticles (A) at 0.2 mg/mL beads.

### (2) Preparation of Internal Standard Peptide

A peptide derived from the myosin motor structure (as a peptide derived from the N-terminal structure; ALELDPNLYR; the region of residue positions 760 to 769 of SEQ ID NO:4) and a peptide derived from the coiled-coil structure (as a peptide derived from the C-terminal structure; LEVNMQALK; the region of residue positions 1572 to 1580 of SEQ ID NO:4) were selected as target peptides, and the peptides labeled with stable isotopes (¹³C and ¹⁵N) were synthesized (AQUA peptide; Sigma-Aldrich).

### (3) Preparation of Measurement Sample

Among the patient samples used in the Section 1, plasma samples of several patients who showed relatively high values of the plasma MYH11 level were pooled to provide a sample to be used as a patient sample. Plasma samples from six Japanese healthy individuals were pooled to provide a healthy individual sample to be used as a negative control.

For immune reaction using the magnetic microparticles, (A) was suspended in a solution prepared by dilution of the sample with PBST, and the reaction was allowed to proceed with stirring for 3 hours at room temperature. Using 0.1 N hydrochloric acid supplemented with 8 M urea as an eluent, an eluate (B) was obtained. (B) was neutralized with 1 M Tris, and then subjected to reductive alkylation according to a conventional method. Trypsin was added thereto to a final concentration of 5.0 µg/mL, and the resulting mixture was incubated at 37°C for 17 hours to obtain a protein digestion product (C). To the resulting solution of (C), the internal standard peptides (10 fmol) were added, and the resulting mixture was subjected to desalting/concentration with GL-Tip SDB (GL Science) to obtain an MRM measurement sample (D).

### (4) Obtaining Peptide Information by Multiple Reaction Monitoring (MRM) Method

The MRM measurement sample (D) obtained was subjected to a triple quadrupole-type mass spectrometer QTRAP 5500 (AB SCIEX) to perform measurement of the two peptides, MYH11-myosin motor peptide and MYH11-coiled-coil peptide. The MRM methods for detection and quantification of these peptides are shown in Table 1. The measurement results were analyzed with Skyline software. Signal reliability was evaluated by confirming that the elution time of the MRM signal in the quantitation channel (Quantitation) accorded with that of the corresponding signal in the verification channel (Verification). Subsequently, in the quantitation channel for each peptide, the peak area ratio of the Light peptide to the Heavy peptide was calculated to calculate the molar concentrations of these peptides contained in the analysis sample.

**[Table 1]**

| Peptide Name | Position | Sequence | Label | | MRM Tran | sitions | |
|---|---|---|---|---|---|---|---|
| | | | | Q1 | CE(V) | Q3 | Analysis |
| MYH11-Myosin Motor | 760-769 | ALELDPNLYR | Light | 602.3 (2+) | | 890.5 (y7+) | Verification |
| | | | | | | 777.4 (y6+) | Quantitation |
| | | | Arg10: Heavy (+10) | 607.3(2+) | 30.5 | 900.5 (y7+) | Verification |
| | | | | | | 787.4(y6+) | Quantitation |
| MYH11-Coiled-Coil | 1572-1580 | LEVNMQALK | Light | 523,3 (2+) | | 803.4 (y7+) | Verification |
| | | | | | | 704.4 (y6+) | Quantitation |
| | | | Lys9: Heavy (+8) | 526.3 (2+) | 27.7 | 809.5 (y7+) 710.4 (y6+) | Verification Quantitation |

### <Results>

Using the internal standard peptides as indices, the molar concentration of MYH11 present in the sample was measured from the quantitative channels for the two target peptides. The results are shown in Fig. 7. In the healthy individual sample used for comparison, the concentrations of both peptides were below the detection limit. On the other hand, in the arteriosclerosis sample, the peptide derived from the C-terminal structure of MYH11 was detected at a very high level, and this level was found to be significantly higher than in the healthy individuals. Regarding the peptide derived from the N-terminal structure, it was revealed that, similarly to the peptide derived from the C-terminal structure, the peptide derived from the N-terminal structure was detected in the arteriosclerosis sample at a significantly higher level than in the healthy individuals, although its level was lower than that of the peptide derived from the C-terminal structure.

### 5. Association of Blood MYH11 Level with Prognosis of Arteriosclerotic Disease

The postoperative course of patients who received PCI or EVT was monitored (monitoring period, 1 to 2.5 years). As a result, three cases of death were found. Since these cases of death did not occur immediately after the intervention, it is apparent that they were not caused by the intervention procedure.
1. Died of infection 64 days after EVT intervention
2. Died of infection 294 days after EVT intervention
3. Died of disseminated intravascular coagulation (DIC) 163 days after PCI intervention

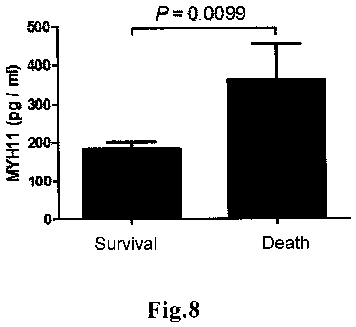

The blood MYH11 level before the intervention, immediately after the intervention, or within several days after the intervention was compared between the death group and the surviving patient group. As a result, as shown in Fig. 8, the blood MYH11 level was higher in the death group. It is known that the risk of severe infection is elevated in peripheral arterial diseases caused by stenosis or occlusion of a peripheral artery (https://www.nhlbi.nih.gov/health/health-topics/topics/atherosclerosis). Further, DIC is a disease state that may occur due to various underlying diseases, and coronary stenosis requiring intervention is one of the underlying diseases of DIC. This data suggests that the blood MYH11 level is useful also as an indicator of prognosis of arteriosclerotic diseases.

## Claims

1. A method for detecting arteriosclerosis, comprising measuring the myosin heavy chain 11 level in a blood sample isolated from a subject, wherein a myosin heavy chain 11 level higher than that in a healthy individual is indicative of the presence of arteriosclerosis in the subject.

2. Use of an anti-myosin heavy chain 11 antibody or an antigen-binding fragment thereof for detecting arteriosclerosis in a blood sample isolated from a subject, wherein a myosin heavy chain 11 level higher than that in a healthy individual is indicative of the presence of arteriosclerosis in the subject.

## Patentansprüche

1. Verfahren zur Detektion von Atherosklerose, welches das Messen des Myosin-Schwerketten-11-Spiegels in einer aus einem Individuum isolierten Blutprobe umfasst, wobei ein Myosin-Schwerketten-11-Spiegel, der höher als bei einem gesunden Individuum ist, indikativ für das Vorliegen von Atherosklerose bei dem Individuum ist.

2. Verwendung eines Anti-Myosin-Schwerketten-11-Antikörpers oder eines Antigenbindenden Fragments davon zur Detektion von Atherosklerose in einer aus einem Individuum isolierten Blutprobe, wobei ein Myosin-Schwerketten-11-Spiegel, der höher als bei einem gesunden Individuum ist, indikativ für das Vorliegen von Atherosklerose bei dem Individuum ist.

## Revendications

1. Procédé pour détecter une artériosclérose, comprenant la mesure du niveau de chaîne lourde de myosine 11 dans un échantillon de sang isolé d'un sujet, dans lequel un niveau de chaîne lourde de myosine 11 supérieur à celui d'un individu sain est indicatif de la présence d'une artériosclérose chez le sujet.

2. Utilisation d'un anticorps anti-chaîne lourde de myosine 11 ou d'un fragment se liant à un antigène de celui-ci pour détecter une artériosclérose dans un échantillon de sang isolé d'un sujet, dans laquelle un niveau de chaîne lourde de myosine 11 supérieur à celui d'un individu sain est indicatif de la présence d'une artériosclérose chez le sujet.


### SEQUENCE LISTING

<110> Public University Corporation Yokohama City University TOSOH Corporation
<120> Blood biomarker for detection of arteriosclerosis
<130> PF588-PCT
<160> 4
<170> PatentIn version 3.5
<210> 1
   <211> 1972
   <212> PRT
   <213> Homo sapiens
<400> 1
   Met Ala Gln Lys Gly Gln Leu Ser Asp Asp Glu Lys Phe Leu Phe Val
   1               5                   10                  15
   Asp Lys Asn Phe Ile Asn Ser Pro Val Ala Gln Ala Asp Trp Ala Ala
               20                  25                  30
   Lys Arg Leu Val Trp Val Pro Ser Glu Lys Gln Gly Phe Glu Ala Ala
           35                  40                  45
   Ser Ile Lys Glu Glu Lys Gly Asp Glu Val Val Val Glu Leu Val Glu
       50                  55                  60
   Asn Gly Lys Lys Val Thr Val Gly Lys Asp Asp Ile Gln Lys Met Asn
   65                  70                  75                  80
   Pro Pro Lys Phe Ser Lys Val Glu Asp Met Ala Glu Leu Thr Cys Leu
                   85                  90                  95
   Asn Glu Ala Ser Val Leu His Asn Leu Arg Glu Arg Tyr Phe Ser Gly
               100                 105                 110
   Leu Ile Tyr Thr Tyr Ser Gly Leu Phe Cys Val Val Val Asn Pro Tyr
           115                 120                 125
   Lys His Leu Pro Ile Tyr Ser Glu Lys Ile Val Asp Met Tyr Lys Gly
       130                 135                 140
   Lys Lys Arg His Glu Met Pro Pro His Ile Tyr Ala Ile Ala Asp Thr
   145                 150                 155                 160
   Ala Tyr Arg Ser Met Leu Gln Asp Arg Glu Asp Gln Ser Ile Leu Cys
                   165                 170                 175
   Thr Gly Glu Ser Gly Ala Gly Lys Thr Glu Asn Thr Lys Lys Val Ile
               180                 185                 190
   Gln Tyr Leu Ala Val Val Ala Ser Ser His Lys Gly Lys Lys Asp Thr
           195                 200                 205
   Ser Ile Thr Gly Glu Leu Glu Lys Gln Leu Leu Gln Ala Asn Pro Ile
       210                 215                 220
   Leu Glu Ala Phe Gly Asn Ala Lys Thr Val Lys Asn Asp Asn Ser Ser
   225                 230                 235                 240
   Arg Phe Gly Lys Phe Ile Arg Ile Asn Phe Asp Val Thr Gly Tyr Ile
                   245                 250                 255
   Val Gly Ala Asn Ile Glu Thr Tyr Leu Leu Glu Lys Ser Arg Ala Ile
               260                 265                 270
   Arg Gln Ala Arg Asp Glu Arg Thr Phe His Ile Phe Tyr Tyr Met Ile
           275                 280                 285
   Ala Gly Ala Lys Glu Lys Met Arg Ser Asp Leu Leu Leu Glu Gly Phe
       290                 295                 300
   Asn Asn Tyr Thr Phe Leu Ser Asn Gly Phe Val Pro Ile Pro Ala Ala
   305                 310                 315                 320
   Gln Asp Asp Glu Met Phe Gln Glu Thr Val Glu Ala Met Ala Ile Met
                   325                 330                 335
   Gly Phe Ser Glu Glu Glu Gln Leu Ser Ile Leu Lys Val Val Ser Ser
               340                 345                 350
   Val Leu Gln Leu Gly Asn Ile Val Phe Lys Lys Glu Arg Asn Thr Asp
           355                 360                 365
   Gln Ala Ser Met Pro Asp Asn Thr Ala Ala Gln Lys Val Cys His Leu
       370                 375                 380
   Met Gly Ile Asn Val Thr Asp Phe Thr Arg Ser Ile Leu Thr Pro Arg
   385                 390                 395                 400
   Ile Lys Val Gly Arg Asp Val Val Gln Lys Ala Gln Thr Lys Glu Gln
                   405                 410                 415
   Ala Asp Phe Ala Val Glu Ala Leu Ala Lys Ala Thr Tyr Glu Arg Leu
               420                 425                 430
   Phe Arg Trp Ile Leu Thr Arg Val Asn Lys Ala Leu Asp Lys Thr His
           435                 440                 445
   Arg Gln Gly Ala Ser Phe Leu Gly Ile Leu Asp Ile Ala Gly Phe Glu
       450                 455                 460
   Ile Phe Glu Val Asn Ser Phe Glu Gln Leu Cys Ile Asn Tyr Thr Asn
   465                 470                 475                 480
   Glu Lys Leu Gln Gln Leu Phe Asn His Thr Met Phe Ile Leu Glu Gln
                   485                 490                 495
   Glu Glu Tyr Gln Arg Glu Gly Ile Glu Trp Asn Phe Ile Asp Phe Gly
               500                 505                 510
   Leu Asp Leu Gln Pro Cys Ile Glu Leu Ile Glu Arg Pro Asn Asn Pro
           515                 520                 525
   Pro Gly Val Leu Ala Leu Leu Asp Glu Glu Cys Trp Phe Pro Lys Ala
       530                 535                 540
   Thr Asp Lys Ser Phe Val Glu Lys Leu Cys Thr Glu Gln Gly Ser His
   545                 550                 555                 560
   Pro Lys Phe Gln Lys Pro Lys Gln Leu Lys Asp Lys Thr Glu Phe Ser
                   565                 570                 575
   Ile Ile His Tyr Ala Gly Lys Val Asp Tyr Asn Ala Ser Ala Trp Leu
               580                 585                 590
   Thr Lys Asn Met Asp Pro Leu Asn Asp Asn Val Thr Ser Leu Leu Asn
           595                 600                 605
   Ala Ser Ser Asp Lys Phe Val Ala Asp Leu Trp Lys Asp Val Asp Arg
       610                 615                 620
   Ile Val Gly Leu Asp Gln Met Ala Lys Met Thr Glu Ser Ser Leu Pro
   625                 630                 635                 640
   Ser Ala Ser Lys Thr Lys Lys Gly Met Phe Arg Thr Val Gly Gln Leu
                   645                 650                 655
   Tyr Lys Glu Gln Leu Gly Lys Leu Met Thr Thr Leu Arg Asn Thr Thr
               660                 665                 670
   Pro Asn Phe Val Arg Cys Ile Ile Pro Asn His Glu Lys Arg Ser Gly
               675                 680                 685
   Lys Leu Asp Ala Phe Leu Val Leu Glu Gln Leu Arg Cys Asn Gly Val
       690                 695                 700
   Leu Glu Gly Ile Arg Ile Cys Arg Gln Gly Phe Pro Asn Arg Ile Val
   705                 710                 715                 720
   Phe Gln Glu Phe Arg Gln Arg Tyr Glu Ile Leu Ala Ala Asn Ala Ile
                   725                 730                 735
   Pro Lys Gly Phe Met Asp Gly Lys Gln Ala Cys Ile Leu Met Ile Lys
               740                 745                 750
   Ala Leu Glu Leu Asp Pro Asn Leu Tyr Arg Ile Gly Gln Ser Lys Ile
           755                 760                 765
   Phe Phe Arg Thr Gly Val Leu Ala His Leu Glu Glu Glu Arg Asp Leu
       770                 775                 780
   Lys Ile Thr Asp Val Ile Met Ala Phe Gln Ala Met Cys Arg Gly Tyr
   785                 790                 795                 800
   Leu Ala Arg Lys Ala Phe Ala Lys Arg Gln Gln Gln Leu Thr Ala Met
                   805                 810                 815
   Lys Val Ile Gln Arg Asn Cys Ala Ala Tyr Leu Lys Leu Arg Asn Trp
               820                 825                 830
   Gln Trp Trp Arg Leu Phe Thr Lys Val Lys Pro Leu Leu Gln Val Thr
           835                 840                 845
   Arg Gln Glu Glu Glu Met Gln Ala Lys Glu Asp Glu Leu Gln Lys Thr
       850                 855                 860
   Lys Glu Arg Gln Gln Lys Ala Glu Asn Glu Leu Lys Glu Leu Glu Gln
   865                 870                 875                 880
   Lys His Ser Gln Leu Thr Glu Glu Lys Asn Leu Leu Gln Glu Gln Leu
                   885                 890                 895
   Gln Ala Glu Thr Glu Leu Tyr Ala Glu Ala Glu Glu Met Arg Val Arg
               900                 905                 910
   Leu Ala Ala Lys Lys Gln Glu Leu Glu Glu Ile Leu His Glu Met Glu
           915                 920                 925
   Ala Arg Leu Glu Glu Glu Glu Asp Arg Gly Gln Gln Leu Gln Ala Glu
       930                 935                 940
   Arg Lys Lys Met Ala Gln Gln Met Leu Asp Leu Glu Glu Gln Leu Glu
   945                 950                 955                 960
   Glu Glu Glu Ala Ala Arg Gln Lys Leu Gln Leu Glu Lys Val Thr Ala
                   965                 970                 975
   Glu Ala Lys Ile Lys Lys Leu Glu Asp Glu Ile Leu Val Met Asp Asp
               980                 985                 990
   Gln Asn Asn Lys Leu Ser Lys Glu  Arg Lys Leu Leu Glu  Glu Arg Ile
           995                 1000                1005
   Ser Asp  Leu Thr Thr Asn Leu  Ala Glu Glu Glu Glu  Lys Ala Lys
       1010                1015                1020
   Asn Leu  Thr Lys Leu Lys Asn  Lys His Glu Ser Met  Ile Ser Glu
       1025                1030                1035
   Leu Glu  Val Arg Leu Lys Lys  Glu Glu Lys Ser Arg  Gln Glu Leu
       1040                1045                1050
   Glu Lys  Leu Lys Arg Lys Leu  Glu Gly Asp Ala Ser  Asp Phe His
       1055                1060                1065
   Glu Gln  Ile Ala Asp Leu Gln  Ala Gln Ile Ala Glu  Leu Lys Met
       1070                1075                1080
   Gln Leu  Ala Lys Lys Glu Glu  Glu Leu Gln Ala Ala  Leu Ala Arg
       1085                1090                1095
   Leu Asp  Asp Glu Ile Ala Gln  Lys Asn Asn Ala Leu  Lys Lys Ile
       1100                1105                1110
   Arg Glu  Leu Glu Gly His Ile  Ser Asp Leu Gln Glu  Asp Leu Asp
       1115                1120                1125
   Ser Glu  Arg Ala Ala Arg Asn  Lys Ala Glu Lys Gln  Lys Arg Asp
       1130                1135                1140
   Leu Gly  Glu Glu Leu Glu Ala  Leu Lys Thr Glu Leu  Glu Asp Thr
       1145                1150                1155
   Leu Asp  Ser Thr Ala Thr Gln  Gln Glu Leu Arg Ala  Lys Arg Glu
       1160                1165                1170
   Gln Glu Val Thr Val Leu Lys Lys Ala Leu Asp Glu Glu Thr Arg
   1175 1180 1185
   Ser His Glu Ala Gln Val Gln Glu Met Arg Gln Lys His Ala Gln
   1190 1195 1200
   Ala Val Glu Glu Leu Thr Glu Gln Leu Glu Gln Phe Lys Arg Ala
   1205 1210 1215
   Lys Ala Asn Leu Asp Lys Asn Lys Gln Thr Leu Glu Lys Glu Asn
   1220 1225 1230
   Ala Asp Leu Ala Gly Glu Leu Arg Val Leu Gly Gln Ala Lys Gln
   1235 1240 1245
   Glu Val Glu His Lys Lys Lys Lys Leu Glu Ala Gln Val Gln Glu
   1250 1255 1260
   Leu Gln Ser Lys Cys Ser Asp Gly Glu Arg Ala Arg Ala Glu Leu
   1265 1270 1275
   Asn Asp Lys Val His Lys Leu Gln Asn Glu Val Glu Ser Val Thr
   1280 1285 1290
   Gly Met Leu Asn Glu Ala Glu Gly Lys Ala Ile Lys Leu Ala Lys
   1295 1300 1305
   Asp Val Ala Ser Leu Ser Ser Gln Leu Gln Asp Thr Gln Glu Leu
   1310 1315 1320
   Leu Gln Glu Glu Thr Arg Gln Lys Leu Asn Val Ser Thr Lys Leu
   1325 1330 1335
   Arg Gln Leu Glu Glu Glu Arg Asn Ser Leu Gln Asp Gln Leu Asp
   1340 1345 1350
   Glu Glu Met Glu Ala Lys Gln Asn Leu Glu Arg His Ile Ser Thr
   1355 1360 1365
   Leu Asn Ile Gln Leu Ser Asp Ser Lys Lys Lys Leu Gln Asp Phe
   1370 1375 1380
   Ala Ser Thr Val Glu Ala Leu Glu Glu Gly Lys Lys Arg Phe Gln
   1385 1390 1395
   Lys Glu Ile Glu Asn Leu Thr Gln Gln Tyr Glu Glu Lys Ala Ala
   1400 1405 1410
   Ala Tyr  Asp Lys Leu Glu Lys  Thr Lys Asn Arg Leu  Gln Gln Glu
       1415                 1420                 1425
   Leu Asp  Asp Leu Val Val Asp  Leu Asp Asn Gln Arg  Gln Leu Val
       1430                 1435                 1440
   Ser Asn  Leu Glu Lys Lys Gln  Arg Lys Phe Asp Gln  Leu Leu Ala
       1445                 1450                 1455
   Glu Glu  Lys Asn Ile Ser Ser  Lys Tyr Ala Asp Glu  Arg Asp Arg
       1460                 1465                 1470
   Ala Glu  Ala Glu Ala Arg Glu  Lys Glu Thr Lys Ala  Leu Ser Leu
       1475                 1480                 1485
   Ala Arg  Ala Leu Glu Glu Ala  Leu Glu Ala Lys Glu  Glu Leu Glu
       1490                 1495                 1500
   Arg Thr  Asn Lys Met Leu Lys  Ala Glu Met Glu Asp  Leu Val Ser
       1505                 1510                 1515
   Ser Lys  Asp Asp Val Gly Lys  Asn Val His Glu Leu  Glu Lys Ser
       1520                 1525                 1530
   Lys Arg  Ala Leu Glu Thr Gln  Met Glu Glu Met Lys  Thr Gln Leu
       1535                 1540                 1545
   Glu Glu  Leu Glu Asp Glu Leu  Gln Ala Thr Glu Asp  Ala Lys Leu
       1550                 1555                 1560
   Arg Leu  Glu Val Asn Met Gln  Ala Leu Lys Gly Gln  Phe Glu Arg
       1565                 1570                 1575
   Asp Leu  Gln Ala Arg Asp Glu  Gln Asn Glu Glu Lys  Arg Arg Gln
       1580                 1585                 1590
   Leu Gln  Arg Gln Leu His Glu  Tyr Glu Thr Glu Leu  Glu Asp Glu
       1595                 1600                 1605
   Arg Lys  Gln Arg Ala Leu Ala  Ala Ala Ala Lys Lys  Lys Leu Glu
       1610                 1615                 1620
   Gly Asp  Leu Lys Asp Leu Glu  Leu Gln Ala Asp Ser  Ala Ile Lys
       1625                 1630                 1635
   Gly Arg  Glu Glu Ala Ile Lys  Gln Leu Arg Lys Leu  Gln Ala Gln
   1640 1645 1650
   Met Lys Asp Phe Gln Arg Glu Leu Glu Asp Ala Arg Ala Ser Arg
   1655 1660 1665
   Asp Glu Ile Phe Ala Thr Ala Lys Glu Asn Glu Lys Lys Ala Lys
   1670 1675 1680
   Ser Leu Glu Ala Asp Leu Met Gln Leu Gln Glu Asp Leu Ala Ala
   1685 1690 1695
   Ala Glu Arg Ala Arg Lys Gln Ala Asp Leu Glu Lys Glu Glu Leu
   1700 1705 1710
   Ala Glu Glu Leu Ala Ser Ser Leu Ser Gly Arg Asn Ala Leu Gln
   1715 1720 1725
   Asp Glu Lys Arg Arg Leu Glu Ala Arg Ile Ala Gln Leu Glu Glu
   1730 1735 1740
   Glu Leu Glu Glu Glu Gln Gly Asn Met Glu Ala Met Ser Asp Arg
   1745 1750 1755
   Val Arg Lys Ala Thr Gln Gln Ala Glu Gln Leu Ser Asn Glu Leu
   1760 1765 1770
   Ala Thr Glu Arg Ser Thr Ala Gln Lys Asn Glu Ser Ala Arg Gln
   1775 1780 1785
   Gln Leu Glu Arg Gln Asn Lys Glu Leu Arg Ser Lys Leu His Glu
   1790 1795 1800
   Met Glu Gly Ala Val Lys Ser Lys Phe Lys Ser Thr Ile Ala Ala
   1805 1810 1815
   Leu Glu Ala Lys Ile Ala Gln Leu Glu Glu Gln Val Glu Gln Glu
   1820 1825 1830
   Ala Arg Glu Lys Gln Ala Ala Thr Lys Ser Leu Lys Gln Lys Asp
   1835 1840 1845
   Lys Lys Leu Lys Glu Ile Leu Leu Gln Val Glu Asp Glu Arg Lys
   1850 1855 1860
   Met Ala Glu Gln Tyr Lys Glu Gln Ala Glu Lys Gly Asn Ala Arg
   1865 1870 1875
   Val Lys Gln Leu Lys Arg Gln Leu Glu Glu Ala Glu Glu Glu Ser
       1880                1885                1890
   Gln Arg Ile Asn Ala Asn Arg Arg Lys Leu Gln Arg Glu Leu Asp
       1895                1900                1905
   Glu Ala Thr Glu Ser Asn Glu Ala Met Gly Arg Glu Val Asn Ala
       1910                1915                1920
   Leu Lys Ser Lys Leu Arg Arg Gly Asn Glu Thr Ser Phe Val Pro
       1925                1930                1935
   Ser Arg Arg Ser Gly Gly Arg Arg Val Ile Glu Asn Ala Asp Gly
       1940                1945                1950
   Ser Glu Glu Glu Thr Asp Thr Arg Asp Ala Asp Phe Asn Gly Thr
       1955                1960                1965
   Lys Ala Ser Glu
       1970
<210> 2
   <211> 1979
   <212> PRT
   <213> Homo sapiens
<400> 2
   Met Ala Gln Lys Gly Gln Leu Ser Asp Asp Glu Lys Phe Leu Phe Val
   1               5                   10                  15
   Asp Lys Asn Phe Ile Asn Ser Pro Val Ala Gln Ala Asp Trp Ala Ala
               20                  25                  30
   Lys Arg Leu Val Trp Val Pro Ser Glu Lys Gln Gly Phe Glu Ala Ala
           35                  40                  45
   Ser Ile Lys Glu Glu Lys Gly Asp Glu Val Val Val Glu Leu Val Glu
       50                  55                  60
   Asn Gly Lys Lys Val Thr Val Gly Lys Asp Asp Ile Gln Lys Met Asn
   65                  70                  75                  80
   Pro Pro Lys Phe Ser Lys Val Glu Asp Met Ala Glu Leu Thr Cys Leu
                   85                  90                  95
   Asn Glu Ala Ser Val Leu His Asn Leu Arg Glu Arg Tyr Phe Ser Gly
               100                 105                 110
   Leu Ile Tyr Thr Tyr Ser Gly Leu Phe Cys Val Val Val Asn Pro Tyr
           115                 120                 125
   Lys His Leu Pro Ile Tyr Ser Glu Lys Ile Val Asp Met Tyr Lys Gly
       130                 135                 140
   Lys Lys Arg His Glu Met Pro Pro His Ile Tyr Ala Ile Ala Asp Thr
   145                 150                 155                 160
   Ala Tyr Arg Ser Met Leu Gln Asp Arg Glu Asp Gln Ser Ile Leu Cys
                   165                 170                 175
   Thr Gly Glu Ser Gly Ala Gly Lys Thr Glu Asn Thr Lys Lys Val Ile
               180                 185                 190
   Gln Tyr Leu Ala Val Val Ala Ser Ser His Lys Gly Lys Lys Asp Thr
           195                 200                 205
   Ser Ile Thr Gln Gly Pro Ser Phe Ala Tyr Gly Glu Leu Glu Lys Gln
       210                 215                 220
   Leu Leu Gln Ala Asn Pro Ile Leu Glu Ala Phe Gly Asn Ala Lys Thr
   225                 230                 235                 240
   Val Lys Asn Asp Asn Ser Ser Arg Phe Gly Lys Phe Ile Arg Ile Asn
                   245                 250                 255
   Phe Asp Val Thr Gly Tyr Ile Val Gly Ala Asn Ile Glu Thr Tyr Leu
               260                 265                 270
   Leu Glu Lys Ser Arg Ala Ile Arg Gln Ala Arg Asp Glu Arg Thr Phe
           275                 280                 285
   His Ile Phe Tyr Tyr Met Ile Ala Gly Ala Lys Glu Lys Met Arg Ser
       290                 295                 300
   Asp Leu Leu Leu Glu Gly Phe Asn Asn Tyr Thr Phe Leu Ser Asn Gly
   305                 310                 315                 320
   Phe Val Pro Ile Pro Ala Ala Gln Asp Asp Glu Met Phe Gln Glu Thr
                   325                 330                 335
   Val Glu Ala Met Ala Ile Met Gly Phe Ser Glu Glu Glu Gln Leu Ser
               340                 345                 350
   Ile Leu Lys Val Val Ser Ser Val Leu Gln Leu Gly Asn Ile Val Phe
           355                 360                 365
   Lys Lys Glu Arg Asn Thr Asp Gln Ala Ser Met Pro Asp Asn Thr Ala
       370                 375                 380
   Ala Gln Lys Val Cys His Leu Met Gly Ile Asn Val Thr Asp Phe Thr
   385                 390                 395                 400
   Arg Ser Ile Leu Thr Pro Arg Ile Lys Val Gly Arg Asp Val Val Gln
                   405                 410                 415
   Lys Ala Gln Thr Lys Glu Gln Ala Asp Phe Ala Val Glu Ala Leu Ala
               420                 425                 430
   Lys Ala Thr Tyr Glu Arg Leu Phe Arg Trp Ile Leu Thr Arg Val Asn
           435                 440                 445
   Lys Ala Leu Asp Lys Thr His Arg Gln Gly Ala Ser Phe Leu Gly Ile
       450                 455                 460
   Leu Asp Ile Ala Gly Phe Glu Ile Phe Glu Val Asn Ser Phe Glu Gln
   465                 470                 475                 480
   Leu Cys Ile Asn Tyr Thr Asn Glu Lys Leu Gln Gln Leu Phe Asn His
                   485                 490                 495
   Thr Met Phe Ile Leu Glu Gln Glu Glu Tyr Gln Arg Glu Gly Ile Glu
               500                 505                 510
   Trp Asn Phe Ile Asp Phe Gly Leu Asp Leu Gln Pro Cys Ile Glu Leu
           515                 520                 525
   Ile Glu Arg Pro Asn Asn Pro Pro Gly Val Leu Ala Leu Leu Asp Glu
       530                 535                 540
   Glu Cys Trp Phe Pro Lys Ala Thr Asp Lys Ser Phe Val Glu Lys Leu
   545                 550                 555                 560
   Cys Thr Glu Gln Gly Ser His Pro Lys Phe Gln Lys Pro Lys Gln Leu
                   565                 570                 575
   Lys Asp Lys Thr Glu Phe Ser Ile Ile His Tyr Ala Gly Lys Val Asp
               580                 585                 590
   Tyr Asn Ala Ser Ala Trp Leu Thr Lys Asn Met Asp Pro Leu Asn Asp
           595                 600                 605
   Asn Val Thr Ser Leu Leu Asn Ala Ser Ser Asp Lys Phe Val Ala Asp
       610                 615                 620
   Leu Trp Lys Asp Val Asp Arg Ile Val Gly Leu Asp Gln Met Ala Lys
   625                 630                 635                 640
   Met Thr Glu Ser Ser Leu Pro Ser Ala Ser Lys Thr Lys Lys Gly Met
                   645                 650                 655
   Phe Arg Thr Val Gly Gln Leu Tyr Lys Glu Gln Leu Gly Lys Leu Met
               660                 665                 670
   Thr Thr Leu Arg Asn Thr Thr Pro Asn Phe Val Arg Cys Ile Ile Pro
           675                 680                 685
   Asn His Glu Lys Arg Ser Gly Lys Leu Asp Ala Phe Leu Val Leu Glu
       690                 695                 700
   Gln Leu Arg Cys Asn Gly Val Leu Glu Gly Ile Arg Ile Cys Arg Gln
   705                 710                 715                 720
   Gly Phe Pro Asn Arg Ile Val Phe Gln Glu Phe Arg Gln Arg Tyr Glu
                   725                 730                 735
   Ile Leu Ala Ala Asn Ala Ile Pro Lys Gly Phe Met Asp Gly Lys Gln
               740                 745                 750
   Ala Cys Ile Leu Met Ile Lys Ala Leu Glu Leu Asp Pro Asn Leu Tyr
           755                 760                 765
   Arg Ile Gly Gln Ser Lys Ile Phe Phe Arg Thr Gly Val Leu Ala His
       770                 775                 780
   Leu Glu Glu Glu Arg Asp Leu Lys Ile Thr Asp Val Ile Met Ala Phe
   785                 790                 795                 800
   Gln Ala Met Cys Arg Gly Tyr Leu Ala Arg Lys Ala Phe Ala Lys Arg
                   805                 810                 815
   Gln Gln Gln Leu Thr Ala Met Lys Val Ile Gln Arg Asn Cys Ala Ala
               820                 825                 830
   Tyr Leu Lys Leu Arg Asn Trp Gln Trp Trp Arg Leu Phe Thr Lys Val
           835                 840                 845
   Lys Pro Leu Leu Gln Val Thr Arg Gln Glu Glu Glu Met Gln Ala Lys
       850                 855                 860
   Glu Asp Glu Leu Gln Lys Thr Lys Glu Arg Gln Gln Lys Ala Glu Asn
   865 870 875 880
   Glu Leu Lys Glu Leu Glu Gln Lys His Ser Gln Leu Thr Glu Glu Lys
   885 890 895
   Asn Leu Leu Gln Glu Gln Leu Gln Ala Glu Thr Glu Leu Tyr Ala Glu
   900 905 910
   Ala Glu Glu Met Arg Val Arg Leu Ala Ala Lys Lys Gln Glu Leu Glu
   915 920 925
   Glu Ile Leu His Glu Met Glu Ala Arg Leu Glu Glu Glu Glu Asp Arg
   930 935 940
   Gly Gln Gln Leu Gln Ala Glu Arg Lys Lys Met Ala Gln Gln Met Leu
   945 950 955 960
   Asp Leu Glu Glu Gln Leu Glu Glu Glu Glu Ala Ala Arg Gln Lys Leu
   965 970 975
   Gln Leu Glu Lys Val Thr Ala Glu Ala Lys Ile Lys Lys Leu Glu Asp
   980 985 990
   Glu Ile Leu Val Met Asp Asp Gln Asn Asn Lys Leu Ser Lys Glu Arg
   995 1000 1005
   Lys Leu Leu Glu Glu Arg Ile Ser Asp Leu Thr Thr Asn Leu Ala
   1010 1015 1020
   Glu Glu Glu Glu Lys Ala Lys Asn Leu Thr Lys Leu Lys Asn Lys
   1025 1030 1035
   His Glu Ser Met Ile Ser Glu Leu Glu Val Arg Leu Lys Lys Glu
   1040 1045 1050
   Glu Lys Ser Arg Gln Glu Leu Glu Lys Leu Lys Arg Lys Leu Glu
   1055 1060 1065
   Gly Asp Ala Ser Asp Phe His Glu Gln Ile Ala Asp Leu Gln Ala
   1070 1075 1080
   Gln Ile Ala Glu Leu Lys Met Gln Leu Ala Lys Lys Glu Glu Glu
   1085 1090 1095
   Leu Gln Ala Ala Leu Ala Arg Leu Asp Asp Glu Ile Ala Gln Lys
   1100 1105 1110
   Asn Asn  Ala Leu Lys Lys Ile  Arg Glu Leu Glu Gly  His Ile Ser
       1115                 1120                 1125
   Asp Leu  Gln Glu Asp Leu Asp  Ser Glu Arg Ala Ala  Arg Asn Lys
       1130                 1135                 1140
   Ala Glu  Lys Gln Lys Arg Asp  Leu Gly Glu Glu Leu  Glu Ala Leu
       1145                 1150                 1155
   Lys Thr  Glu Leu Glu Asp Thr  Leu Asp Ser Thr Ala  Thr Gln Gln
       1160                 1165                 1170
   Glu Leu  Arg Ala Lys Arg Glu  Gln Glu Val Thr Val  Leu Lys Lys
       1175                 1180                 1185
   Ala Leu  Asp Glu Glu Thr Arg  Ser His Glu Ala Gln  Val Gln Glu
       1190                 1195                 1200
   Met Arg  Gln Lys His Ala Gln  Ala Val Glu Glu Leu  Thr Glu Gln
       1205                 1210                 1215
   Leu Glu  Gln Phe Lys Arg Ala  Lys Ala Asn Leu Asp  Lys Asn Lys
       1220                 1225                 1230
   Gln Thr  Leu Glu Lys Glu Asn  Ala Asp Leu Ala Gly  Glu Leu Arg
       1235                 1240                 1245
   Val Leu  Gly Gln Ala Lys Gln  Glu Val Glu His Lys  Lys Lys Lys
       1250                 1255                 1260
   Leu Glu  Ala Gln Val Gln Glu  Leu Gln Ser Lys Cys  Ser Asp Gly
       1265                 1270                 1275
   Glu Arg  Ala Arg Ala Glu Leu  Asn Asp Lys Val His  Lys Leu Gln
       1280                 1285                 1290
   Asn Glu  Val Glu Ser Val Thr  Gly Met Leu Asn Glu  Ala Glu Gly
       1295                 1300                 1305
   Lys Ala  Ile Lys Leu Ala Lys  Asp Val Ala Ser Leu  Ser Ser Gln
       1310                 1315                 1320
   Leu Gln  Asp Thr Gln Glu Leu  Leu Gln Glu Glu Thr  Arg Gln Lys
       1325                 1330                 1335
   Leu Asn  Val Ser Thr Lys Leu  Arg Gln Leu Glu Glu  Glu Arg Asn
       1340                 1345                 1350
   Ser Leu  Gln Asp Gln Leu Asp  Glu Glu Met Glu Ala  Lys Gln Asn
       1355                 1360                 1365
   Leu Glu  Arg His Ile Ser Thr  Leu Asn Ile Gln Leu  Ser Asp Ser
       1370                 1375                 1380
   Lys Lys  Lys Leu Gln Asp Phe  Ala Ser Thr Val Glu  Ala Leu Glu
       1385                 1390                 1395
   Glu Gly  Lys Lys Arg Phe Gln  Lys Glu Ile Glu Asn  Leu Thr Gln
       1400                 1405                 1410
   Gln Tyr  Glu Glu Lys Ala Ala  Ala Tyr Asp Lys Leu  Glu Lys Thr
       1415                 1420                 1425
   Lys Asn  Arg Leu Gln Gln Glu  Leu Asp Asp Leu Val  Val Asp Leu
       1430                 1435                 1440
   Asp Asn  Gln Arg Gln Leu Val  Ser Asn Leu Glu Lys  Lys Gln Arg
       1445                 1450                 1455
   Lys Phe  Asp Gln Leu Leu Ala  Glu Glu Lys Asn Ile  Ser Ser Lys
       1460                 1465                 1470
   Tyr Ala  Asp Glu Arg Asp Arg  Ala Glu Ala Glu Ala  Arg Glu Lys
       1475                 1480                 1485
   Glu Thr  Lys Ala Leu Ser Leu  Ala Arg Ala Leu Glu  Glu Ala Leu
       1490                 1495                 1500
   Glu Ala  Lys Glu Glu Leu Glu  Arg Thr Asn Lys Met  Leu Lys Ala
       1505                 1510                 1515
   Glu Met  Glu Asp Leu Val Ser  Ser Lys Asp Asp Val  Gly Lys Asn
       1520                 1525                 1530
   Val His  Glu Leu Glu Lys Ser  Lys Arg Ala Leu Glu  Thr Gln Met
       1535                 1540                 1545
   Glu Glu  Met Lys Thr Gln Leu  Glu Glu Leu Glu Asp  Glu Leu Gln
       1550                 1555                 1560
   Ala Thr  Glu Asp Ala Lys Leu  Arg Leu Glu Val Asn  Met Gln Ala
       1565                 1570                 1575
   Leu Lys  Gly Gln Phe Glu Arg  Asp Leu Gln Ala Arg  Asp Glu Gln
       1580                 1585                 1590
   Asn Glu  Glu Lys Arg Arg Gln  Leu Gln Arg Gln Leu  His Glu Tyr
       1595                 1600                 1605
   Glu Thr  Glu Leu Glu Asp Glu  Arg Lys Gln Arg Ala  Leu Ala Ala
       1610                 1615                 1620
   Ala Ala  Lys Lys Lys Leu Glu  Gly Asp Leu Lys Asp  Leu Glu Leu
       1625                 1630                 1635
   Gln Ala  Asp Ser Ala Ile Lys  Gly Arg Glu Glu Ala  Ile Lys Gln
       1640                 1645                 1650
   Leu Arg  Lys Leu Gln Ala Gln  Met Lys Asp Phe Gln  Arg Glu Leu
       1655                 1660                 1665
   Glu Asp  Ala Arg Ala Ser Arg  Asp Glu Ile Phe Ala  Thr Ala Lys
       1670                 1675                 1680
   Glu Asn  Glu Lys Lys Ala Lys  Ser Leu Glu Ala Asp  Leu Met Gln
       1685                 1690                 1695
   Leu Gln  Glu Asp Leu Ala Ala  Ala Glu Arg Ala Arg  Lys Gln Ala
       1700                 1705                 1710
   Asp Leu  Glu Lys Glu Glu Leu  Ala Glu Glu Leu Ala  Ser Ser Leu
       1715                 1720                 1725
   Ser Gly  Arg Asn Ala Leu Gln  Asp Glu Lys Arg Arg  Leu Glu Ala
       1730                 1735                 1740
   Arg Ile  Ala Gln Leu Glu Glu  Glu Leu Glu Glu Glu  Gln Gly Asn
       1745                 1750                 1755
   Met Glu  Ala Met Ser Asp Arg  Val Arg Lys Ala Thr  Gln Gln Ala
       1760                 1765                 1770
   Glu Gln  Leu Ser Asn Glu Leu  Ala Thr Glu Arg Ser  Thr Ala Gln
       1775                 1780                 1785
   Lys Asn  Glu Ser Ala Arg Gln  Gln Leu Glu Arg Gln  Asn Lys Glu
       1790                 1795                 1800
   Leu Arg  Ser Lys Leu His Glu  Met Glu Gly Ala Val  Lys Ser Lys
       1805                 1810                 1815
   Phe Lys  Ser Thr Ile Ala Ala  Leu Glu Ala Lys Ile  Ala Gln Leu
   1820 1825 1830
   Glu Glu Gln Val Glu Gln Glu Ala Arg Glu Lys Gln Ala Ala Thr
   1835 1840 1845
   Lys Ser Leu Lys Gln Lys Asp Lys Lys Leu Lys Glu Ile Leu Leu
   1850 1855 1860
   Gln Val Glu Asp Glu Arg Lys Met Ala Glu Gln Tyr Lys Glu Gln
   1865 1870 1875
   Ala Glu Lys Gly Asn Ala Arg Val Lys Gln Leu Lys Arg Gln Leu
   1880 1885 1890
   Glu Glu Ala Glu Glu Glu Ser Gln Arg Ile Asn Ala Asn Arg Arg
   1895 1900 1905
   Lys Leu Gln Arg Glu Leu Asp Glu Ala Thr Glu Ser Asn Glu Ala
   1910 1915 1920
   Met Gly Arg Glu Val Asn Ala Leu Lys Ser Lys Leu Arg Arg Gly
   1925 1930 1935
   Asn Glu Thr Ser Phe Val Pro Ser Arg Arg Ser Gly Gly Arg Arg
   1940 1945 1950
   Val Ile Glu Asn Ala Asp Gly Ser Glu Glu Glu Thr Asp Thr Arg
   1955 1960 1965
   Asp Ala Asp Phe Asn Gly Thr Lys Ala Ser Glu
   1970 1975
<210> 3
   <211> 1938
   <212> PRT
   <213> Homo sapiens
<400> 3
   Met Ala Gln Lys Gly Gln Leu Ser Asp Asp Glu Lys Phe Leu Phe Val
   1               5                   10                  15
   Asp Lys Asn Phe Ile Asn Ser Pro Val Ala Gln Ala Asp Trp Ala Ala
               20                  25                  30
   Lys Arg Leu Val Trp Val Pro Ser Glu Lys Gln Gly Phe Glu Ala Ala
           35                  40                  45
   Ser Ile Lys Glu Glu Lys Gly Asp Glu Val Val Val Glu Leu Val Glu
   50 55 60
   Asn Gly Lys Lys Val Thr Val Gly Lys Asp Asp Ile Gln Lys Met Asn
   65 70 75 80
   Pro Pro Lys Phe Ser Lys Val Glu Asp Met Ala Glu Leu Thr Cys Leu
   85 90 95
   Asn Glu Ala Ser Val Leu His Asn Leu Arg Glu Arg Tyr Phe Ser Gly
   100 105 110
   Leu Ile Tyr Thr Tyr Ser Gly Leu Phe Cys Val Val Val Asn Pro Tyr
   115 120 125
   Lys His Leu Pro Ile Tyr Ser Glu Lys Ile Val Asp Met Tyr Lys Gly
   130 135 140
   Lys Lys Arg His Glu Met Pro Pro His Ile Tyr Ala Ile Ala Asp Thr
   145 150 155 160
   Ala Tyr Arg Ser Met Leu Gln Asp Arg Glu Asp Gln Ser Ile Leu Cys
   165 170 175
   Thr Gly Glu Ser Gly Ala Gly Lys Thr Glu Asn Thr Lys Lys Val Ile
   180 185 190
   Gln Tyr Leu Ala Val Val Ala Ser Ser His Lys Gly Lys Lys Asp Thr
   195 200 205
   Ser Ile Thr Gly Glu Leu Glu Lys Gln Leu Leu Gln Ala Asn Pro Ile
   210 215 220
   Leu Glu Ala Phe Gly Asn Ala Lys Thr Val Lys Asn Asp Asn Ser Ser
   225 230 235 240
   Arg Phe Gly Lys Phe Ile Arg Ile Asn Phe Asp Val Thr Gly Tyr Ile
   245 250 255
   Val Gly Ala Asn Ile Glu Thr Tyr Leu Leu Glu Lys Ser Arg Ala Ile
   260 265 270
   Arg Gln Ala Arg Asp Glu Arg Thr Phe His Ile Phe Tyr Tyr Met Ile
   275 280 285
   Ala Gly Ala Lys Glu Lys Met Arg Ser Asp Leu Leu Leu Glu Gly Phe
   290 295 300
   Asn Asn Tyr Thr Phe Leu Ser Asn Gly Phe Val Pro Ile Pro Ala Ala
   305                 310                 315                 320
   Gln Asp Asp Glu Met Phe Gln Glu Thr Val Glu Ala Met Ala Ile Met
                   325                 330                 335
   Gly Phe Ser Glu Glu Glu Gln Leu Ser Ile Leu Lys Val Val Ser Ser
               340                 345                 350
   Val Leu Gln Leu Gly Asn Ile Val Phe Lys Lys Glu Arg Asn Thr Asp
           355                 360                 365
   Gln Ala Ser Met Pro Asp Asn Thr Ala Ala Gln Lys Val Cys His Leu
       370                 375                 380
   Met Gly Ile Asn Val Thr Asp Phe Thr Arg Ser Ile Leu Thr Pro Arg
   385                 390                 395                 400
   Ile Lys Val Gly Arg Asp Val Val Gln Lys Ala Gln Thr Lys Glu Gln
                   405                 410                 415
   Ala Asp Phe Ala Val Glu Ala Leu Ala Lys Ala Thr Tyr Glu Arg Leu
               420                 425                 430
   Phe Arg Trp Ile Leu Thr Arg Val Asn Lys Ala Leu Asp Lys Thr His
           435                 440                 445
   Arg Gln Gly Ala Ser Phe Leu Gly Ile Leu Asp Ile Ala Gly Phe Glu
       450                 455                 460
   Ile Phe Glu Val Asn Ser Phe Glu Gln Leu Cys Ile Asn Tyr Thr Asn
   465                 470                 475                 480
   Glu Lys Leu Gln Gln Leu Phe Asn His Thr Met Phe Ile Leu Glu Gln
                   485                 490                 495
   Glu Glu Tyr Gln Arg Glu Gly Ile Glu Trp Asn Phe Ile Asp Phe Gly
               500                 505                 510
   Leu Asp Leu Gln Pro Cys Ile Glu Leu Ile Glu Arg Pro Asn Asn Pro
           515                 520                 525
   Pro Gly Val Leu Ala Leu Leu Asp Glu Glu Cys Trp Phe Pro Lys Ala
       530                 535                 540
   Thr Asp Lys Ser Phe Val Glu Lys Leu Cys Thr Glu Gln Gly Ser His
   545                 550                 555                 560
   Pro Lys Phe Gln Lys Pro Lys Gln Leu Lys Asp Lys Thr Glu Phe Ser
                   565                 570                 575
   Ile Ile His Tyr Ala Gly Lys Val Asp Tyr Asn Ala Ser Ala Trp Leu
               580                 585                 590
   Thr Lys Asn Met Asp Pro Leu Asn Asp Asn Val Thr Ser Leu Leu Asn
           595                 600                 605
   Ala Ser Ser Asp Lys Phe Val Ala Asp Leu Trp Lys Asp Val Asp Arg
       610                 615                 620
   Ile Val Gly Leu Asp Gln Met Ala Lys Met Thr Glu Ser Ser Leu Pro
   625                 630                 635                 640
   Ser Ala Ser Lys Thr Lys Lys Gly Met Phe Arg Thr Val Gly Gln Leu
                   645                 650                 655
   Tyr Lys Glu Gln Leu Gly Lys Leu Met Thr Thr Leu Arg Asn Thr Thr
               660                 665                 670
   Pro Asn Phe Val Arg Cys Ile Ile Pro Asn His Glu Lys Arg Ser Gly
           675                 680                 685
   Lys Leu Asp Ala Phe Leu Val Leu Glu Gln Leu Arg Cys Asn Gly Val
       690                 695                 700
   Leu Glu Gly Ile Arg Ile Cys Arg Gln Gly Phe Pro Asn Arg Ile Val
   705                 710                 715                 720
   Phe Gln Glu Phe Arg Gln Arg Tyr Glu Ile Leu Ala Ala Asn Ala Ile
                   725                 730                 735
   Pro Lys Gly Phe Met Asp Gly Lys Gln Ala Cys Ile Leu Met Ile Lys
               740                 745                 750
   Ala Leu Glu Leu Asp Pro Asn Leu Tyr Arg Ile Gly Gln Ser Lys Ile
           755                 760                 765
   Phe Phe Arg Thr Gly Val Leu Ala His Leu Glu Glu Glu Arg Asp Leu
       770                 775                 780
   Lys Ile Thr Asp Val Ile Met Ala Phe Gln Ala Met Cys Arg Gly Tyr
   785                 790                 795                 800
   Leu Ala Arg Lys Ala Phe Ala Lys Arg Gln Gln Gln Leu Thr Ala Met
                   805                 810                 815
   Lys Val Ile Gln Arg Asn Cys Ala Ala Tyr Leu Lys Leu Arg Asn Trp
   820 825 830
   Gln Trp Trp Arg Leu Phe Thr Lys Val Lys Pro Leu Leu Gln Val Thr
   835 840 845
   Arg Gln Glu Glu Glu Met Gln Ala Lys Glu Asp Glu Leu Gln Lys Thr
   850 855 860
   Lys Glu Arg Gln Gln Lys Ala Glu Asn Glu Leu Lys Glu Leu Glu Gln
   865 870 875 880
   Lys His Ser Gln Leu Thr Glu Glu Lys Asn Leu Leu Gln Glu Gln Leu
   885 890 895
   Gln Ala Glu Thr Glu Leu Tyr Ala Glu Ala Glu Glu Met Arg Val Arg
   900 905 910
   Leu Ala Ala Lys Lys Gln Glu Leu Glu Glu Ile Leu His Glu Met Glu
   915 920 925
   Ala Arg Leu Glu Glu Glu Glu Asp Arg Gly Gln Gln Leu Gln Ala Glu
   930 935 940
   Arg Lys Lys Met Ala Gln Gln Met Leu Asp Leu Glu Glu Gln Leu Glu
   945 950 955 960
   Glu Glu Glu Ala Ala Arg Gln Lys Leu Gln Leu Glu Lys Val Thr Ala
   965 970 975
   Glu Ala Lys Ile Lys Lys Leu Glu Asp Glu Ile Leu Val Met Asp Asp
   980 985 990
   Gln Asn Asn Lys Leu Ser Lys Glu Arg Lys Leu Leu Glu Glu Arg Ile
   995 1000 1005
   Ser Asp Leu Thr Thr Asn Leu Ala Glu Glu Glu Glu Lys Ala Lys
   1010 1015 1020
   Asn Leu Thr Lys Leu Lys Asn Lys His Glu Ser Met Ile Ser Glu
   1025 1030 1035
   Leu Glu Val Arg Leu Lys Lys Glu Glu Lys Ser Arg Gln Glu Leu
   1040 1045 1050
   Glu Lys Leu Lys Arg Lys Leu Glu Gly Asp Ala Ser Asp Phe His
   1055 1060 1065
   Glu Gln Ile Ala Asp Leu Gln Ala Gln Ile Ala Glu Leu Lys Met
   1070 1075 1080
   Gln Leu Ala Lys Lys Glu Glu Glu Leu Gln Ala Ala Leu Ala Arg
   1085 1090 1095
   Leu Asp Asp Glu Ile Ala Gln Lys Asn Asn Ala Leu Lys Lys Ile
   1100 1105 1110
   Arg Glu Leu Glu Gly His Ile Ser Asp Leu Gln Glu Asp Leu Asp
   1115 1120 1125
   Ser Glu Arg Ala Ala Arg Asn Lys Ala Glu Lys Gln Lys Arg Asp
   1130 1135 1140
   Leu Gly Glu Glu Leu Glu Ala Leu Lys Thr Glu Leu Glu Asp Thr
   1145 1150 1155
   Leu Asp Ser Thr Ala Thr Gln Gln Glu Leu Arg Ala Lys Arg Glu
   1160 1165 1170
   Gln Glu Val Thr Val Leu Lys Lys Ala Leu Asp Glu Glu Thr Arg
   1175 1180 1185
   Ser His Glu Ala Gln Val Gln Glu Met Arg Gln Lys His Ala Gln
   1190 1195 1200
   Ala Val Glu Glu Leu Thr Glu Gln Leu Glu Gln Phe Lys Arg Ala
   1205 1210 1215
   Lys Ala Asn Leu Asp Lys Asn Lys Gln Thr Leu Glu Lys Glu Asn
   1220 1225 1230
   Ala Asp Leu Ala Gly Glu Leu Arg Val Leu Gly Gln Ala Lys Gln
   1235 1240 1245
   Glu Val Glu His Lys Lys Lys Lys Leu Glu Ala Gln Val Gln Glu
   1250 1255 1260
   Leu Gln Ser Lys Cys Ser Asp Gly Glu Arg Ala Arg Ala Glu Leu
   1265 1270 1275
   Asn Asp Lys Val His Lys Leu Gln Asn Glu Val Glu Ser Val Thr
   1280 1285 1290
   Gly Met  Leu Asn Glu Ala Glu  Gly Lys Ala Ile Lys  Leu Ala Lys
       1295                 1300                 1305
   Asp Val  Ala Ser Leu Ser Ser  Gln Leu Gln Asp Thr  Gln Glu Leu
       1310                 1315                 1320
   Leu Gln  Glu Glu Thr Arg Gln  Lys Leu Asn Val Ser  Thr Lys Leu
       1325                 1330                 1335
   Arg Gln  Leu Glu Glu Glu Arg  Asn Ser Leu Gln Asp  Gln Leu Asp
       1340                 1345                 1350
   Glu Glu  Met Glu Ala Lys Gln  Asn Leu Glu Arg His  Ile Ser Thr
       1355                 1360                 1365
   Leu Asn  Ile Gln Leu Ser Asp  Ser Lys Lys Lys Leu  Gln Asp Phe
       1370                 1375                 1380
   Ala Ser  Thr Val Glu Ala Leu  Glu Glu Gly Lys Lys  Arg Phe Gln
       1385                 1390                 1395
   Lys Glu  Ile Glu Asn Leu Thr  Gln Gln Tyr Glu Glu  Lys Ala Ala
       1400                 1405                 1410
   Ala Tyr  Asp Lys Leu Glu Lys  Thr Lys Asn Arg Leu  Gln Gln Glu
       1415                 1420                 1425
   Leu Asp  Asp Leu Val Val Asp  Leu Asp Asn Gln Arg  Gln Leu Val
       1430                 1435                 1440
   Ser Asn  Leu Glu Lys Lys Gln  Arg Lys Phe Asp Gln  Leu Leu Ala
       1445                 1450                 1455
   Glu Glu  Lys Asn Ile Ser Ser  Lys Tyr Ala Asp Glu  Arg Asp Arg
       1460                 1465                 1470
   Ala Glu  Ala Glu Ala Arg Glu  Lys Glu Thr Lys Ala  Leu Ser Leu
       1475                 1480                 1485
   Ala Arg  Ala Leu Glu Glu Ala  Leu Glu Ala Lys Glu  Glu Leu Glu
       1490                 1495                 1500
   Arg Thr  Asn Lys Met Leu Lys  Ala Glu Met Glu Asp  Leu Val Ser
       1505                 1510                 1515
   Ser Lys  Asp Asp Val Gly Lys  Asn Val His Glu Leu  Glu Lys Ser
       1520                 1525                 1530
   Lys Arg Ala Leu Glu Thr Gln Met Glu Glu Met Lys Thr Gln Leu
   1535 1540 1545
   Glu Glu Leu Glu Asp Glu Leu Gln Ala Thr Glu Asp Ala Lys Leu
   1550 1555 1560
   Arg Leu Glu Val Asn Met Gln Ala Leu Lys Gly Gln Phe Glu Arg
   1565 1570 1575
   Asp Leu Gln Ala Arg Asp Glu Gln Asn Glu Glu Lys Arg Arg Gln
   1580 1585 1590
   Leu Gln Arg Gln Leu His Glu Tyr Glu Thr Glu Leu Glu Asp Glu
   1595 1600 1605
   Arg Lys Gln Arg Ala Leu Ala Ala Ala Ala Lys Lys Lys Leu Glu
   1610 1615 1620
   Gly Asp Leu Lys Asp Leu Glu Leu Gln Ala Asp Ser Ala Ile Lys
   1625 1630 1635
   Gly Arg Glu Glu Ala Ile Lys Gln Leu Arg Lys Leu Gln Ala Gln
   1640 1645 1650
   Met Lys Asp Phe Gln Arg Glu Leu Glu Asp Ala Arg Ala Ser Arg
   1655 1660 1665
   Asp Glu Ile Phe Ala Thr Ala Lys Glu Asn Glu Lys Lys Ala Lys
   1670 1675 1680
   Ser Leu Glu Ala Asp Leu Met Gln Leu Gln Glu Asp Leu Ala Ala
   1685 1690 1695
   Ala Glu Arg Ala Arg Lys Gln Ala Asp Leu Glu Lys Glu Glu Leu
   1700 1705 1710
   Ala Glu Glu Leu Ala Ser Ser Leu Ser Gly Arg Asn Ala Leu Gln
   1715 1720 1725
   Asp Glu Lys Arg Arg Leu Glu Ala Arg Ile Ala Gln Leu Glu Glu
   1730 1735 1740
   Glu Leu Glu Glu Glu Gln Gly Asn Met Glu Ala Met Ser Asp Arg
   1745 1750 1755
   Val Arg Lys Ala Thr Gln Gln Ala Glu Gln Leu Ser Asn Glu Leu
   1760 1765 1770
   Ala Thr Glu Arg Ser Thr Ala Gln Lys Asn Glu Ser Ala Arg Gln
   1775 1780 1785
   Gln Leu Glu Arg Gln Asn Lys Glu Leu Arg Ser Lys Leu His Glu
   1790 1795 1800
   Met Glu Gly Ala Val Lys Ser Lys Phe Lys Ser Thr Ile Ala Ala
   1805 1810 1815
   Leu Glu Ala Lys Ile Ala Gln Leu Glu Glu Gln Val Glu Gln Glu
   1820 1825 1830
   Ala Arg Glu Lys Gln Ala Ala Thr Lys Ser Leu Lys Gln Lys Asp
   1835 1840 1845
   Lys Lys Leu Lys Glu Ile Leu Leu Gln Val Glu Asp Glu Arg Lys
   1850 1855 1860
   Met Ala Glu Gln Tyr Lys Glu Gln Ala Glu Lys Gly Asn Ala Arg
   1865 1870 1875
   Val Lys Gln Leu Lys Arg Gln Leu Glu Glu Ala Glu Glu Glu Ser
   1880 1885 1890
   Gln Arg Ile Asn Ala Asn Arg Arg Lys Leu Gln Arg Glu Leu Asp
   1895 1900 1905
   Glu Ala Thr Glu Ser Asn Glu Ala Met Gly Arg Glu Val Asn Ala
   1910 1915 1920
   Leu Lys Ser Lys Leu Arg Gly Pro Pro Pro Gln Glu Thr Ser Gln
   1925 1930 1935
<210> 4
   <211> 1945
   <212> PRT
   <213> Homo sapiens
<400> 4